(19) Europäisches Patentamt
European Patent Office
Office européen des brevets

(11) **EP 3 786 960 A1**

(12) **EUROPEAN PATENT APPLICATION**
published in accordance with Art. 153(4) EPC

(43) Date of publication:
**03.03.2021 Bulletin 2021/09**

(51) Int Cl.:
***G16B 30/00*** (2019.01)    ***C12M 1/00*** (2006.01)
***C12Q 1/6809*** (2018.01)

(21) Application number: **19791813.9**

(22) Date of filing: **25.03.2019**

(86) International application number:
**PCT/JP2019/012294**

(87) International publication number:
**WO 2019/208052 (31.10.2019 Gazette 2019/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.04.2018 JP 2018084302**

(71) Applicant: **Nonprofit Organization North East
Japan Study Group
Saitama City, Saitama 330-0843 (JP)**

(72) Inventors:
• **HAGIWARA, Koichi
Shimotsuke-shi, Tochigi 329-0498 (JP)**
• **INOUE, Yoshiaki
Kawagoe-shi, Saitama 350-8550 (JP)**

(74) Representative: **Cabinet Beaumont
4, Place Robert Schuman
B.P. 1529
38025 Grenoble Cedex 1 (FR)**

(54) **PROGRAM, INFORMATION PROCESSING METHOD, AND INFORMATION PROCESSING DEVICE**

(57)    The object is to provide a program or the like for determining whether a base sequence is mutation positive or mutation negative taking the probability of occurrence of false positives into account.

A program causes a computer to execute the processing of: acquiring a reference nucleic acid base sequence including a normal sequence and a mutated sequence in which a part of the normal sequence is mutated; acquiring a plurality of sample nucleic acid base sequences read from a sample; determining the number of read sequences corresponding to the reference nucleic acid base sequence and the number of mutated sequences corresponding to the mutated sequence among the sample nucleic acid base sequences; acquiring an error rate obtained when a nucleic acid base sequence is read; and determining whether or not the sample nucleic acid base sequence is mutation positive based on the number of mutated sequences, the number of read sequences, the error rate and a predetermined false positive rate.

FIG.16

EP 3 786 960 A1

**Description**

[Technical Field]

The present invention relates to a program, an information processing method and an information processing apparatus.

[Background Art]

Patent Document 1 discloses a method of obtaining a renal cancer tissue from a patient with renal cancer, measuring an expression level or an expression amount of a gene related to renal cancer and determining the malignancy of renal cancer.

[Prior Art Document]

[Patent Document]

[Patent Document 1] Japanese Patent Application Laid-Open Publication No. 2016-86678

[Summary of the Invention]

[Problems to be Solved by Invention]

A next-generation sequencer is used that reads a large number of genetic base sequences at high speed. The next-generation sequencer achieves faster reading by simultaneously reading multiple base sequences. An appropriate anticancer agent is selected based on the mutations of the base sequences read by using the next-generation sequencer. During the procedure of amplifying genes to read a base sequence, however, some replication errors may occur. During the procedure of reading base sequences by the next-generation sequencer as well, some reading errors may occur.
A false positive determination may occur when a sample in which no cancer cell is present is erroneously determined that cancer cells are present due to replication errors and reading errors in the next-generation sequencer. In the method disclosed in Patent Document 1, determination cannot be made taking the probability of occurrence of a false positive into account.
In one aspect, an object is to provide a program or the like to determine whether a base sequence is mutation positive or mutation negative taking into account the probability of occurrence of false positives due to replication errors and reading errors in the next-generation sequencer.

[Means for Solving Problems]

A program causes a computer to execute the processing of: acquiring a reference nucleic acid base sequence including a normal sequence and a mutated sequence in which a part of the normal sequence is mutated; acquiring a plurality of sample nucleic acid base sequences read from a sample; determining the number of read sequences corresponding to the reference nucleic acid base sequence and the number of mutated sequences corresponding to the mutated sequence from the sample nucleic acid base sequences; acquiring an error rate obtained when a nucleic acid base sequence is read; and determining whether or not the sample nucleic acid base sequence is mutation positive based on the number of mutated sequences, the number of read sequences, the error rate and a predetermined false positive rate.

[Effects of Invention]

In one aspect, it is possible to provide a program or the like to determine whether a base sequence is mutation positive or mutation negative taking the probability of occurrence of false positives into account.

[Brief Description of Drawings]

FIG. 1 illustrates the outline of a therapeutic procedure using genetic testing.

FIG. 2 illustrates the outline of a method for the genetic testing.

FIG. 3 illustrates the outline of the method for the genetic testing.

FIG. 4 illustrates one example of readings.

FIG. 5 illustrates one example of an amplification procedure of base sequences.

FIG. 6 illustrates another example of the amplification procedure of base sequences.

FIG. 7 illustrates a probability density distribution for the number of mutation-positive sequences M related to one target sequence.

FIG. 8 is an enlarged view of a portion A in FIG. 7.

FIG. 9 illustrates the configuration of a genetic testing system.

FIG. 10 illustrates a record layout of a reference sequence DB.

FIG. 11 illustrates one example of a read data file.

FIG. 12 illustrates a record layout of a reading result DB.

FIG. 13 illustrates a record layout of a determination result DB.

FIG. 14 illustrates a record layout of an unknown mutation DB.

FIG. 15 is one example of a screen displaying a determination result.

FIG. 16 is another example of a screen display a determination result.

FIG. 17 is a flowchart showing the flow of the processing of a program.

FIG. 18 is a flowchart showing the flow of processing of a subroutine for determination.

FIG. 19 is a flowchart showing the flow of processing of a subroutine for threshold determination.

FIG. 20 is a flowchart showing the flow of processing of a subroutine for statistic determination.

FIG. 21 is a flowchart showing the flow of processing of a subroutine for first threshold MP calculation.

FIG. 22 is a flowchart showing the flow of processing of a subroutine for second threshold MN calculation.

FIG. 23 illustrates a record layout of a parameter DB.

FIG. 24 is a flowchart showing the flow of processing of a subroutine for threshold calculation according to Embodiment 2.

FIG. 25 is a flowchart showing the flow of processing of a subroutine for parameter adjustment.

FIG. 26 is a flowchart showing the flow of processing of a program according to Embodiment 3.

FIG. 27 is a flowchart showing the flow of processing of the program according to Embodiment 3.

FIG. 28 is a flowchart showing the flow of the program according to Embodiment 3.

FIG. 29 is a functional block diagram of an information processing apparatus.

FIG. 30 illustrates the configuration of a genetic testing system according to Embodiment 5.

[Mode for Carrying out Invention]

[Embodiment 1]

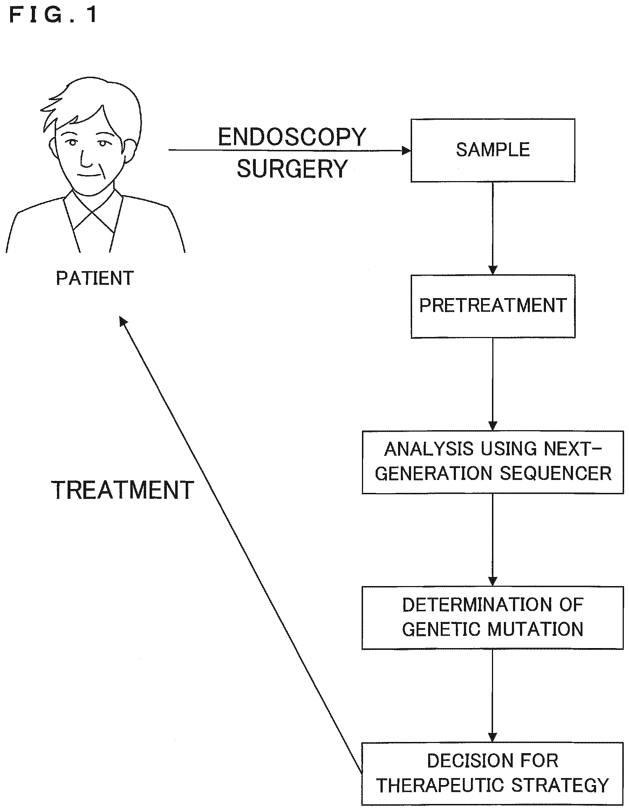

FIG. 1 illustrates the outline of a therapeutic procedure using genetic testing. In the following, treatment for lung cancer will be described as an example. First, a sample is collected from a patient with cancer or a patient suspected of having cancer. A sample is collected by techniques such as brush cytology, cytologic washings or a transbronchial lung biopsy using a bronchoscope, an ultrasound-or CT-guided needle biopsy and so on. These sample collection methods through an endoscope are relatively low invasive but are able to collect only a small amount of samples.

A sample may be obtained by relatively high invasive sample collection methods such as a percutaneous needle biopsy, an open lung needle biopsy, a thoracoscopic lung biopsy and so on. If a surgery like lung resection is performed, a part of the resected tissue is used as a sample for genetic testing.

Then, pretreatment is performed for extracting nucleic acid, i.e., deoxyribonucleic acid (DNA) and ribonucleic acid (RNA) from the sample and amplifying them. The details of the pretreatment will be described later. A next-generation sequencer reads a nucleic acid base sequence, i.e., a DNA base sequence and RNA base sequence. The read nucleic acid base sequence is stored in a file in FASTQ format, for example. In the following description, the nucleic acid base sequence may be referred to as a base sequence.

The stored nucleic acid base sequence is compared with a predetermined reference sequence to determine what type of genetic mutation occurs. Depending on the state of the genetic mutation, a therapeutic strategy such as selection of an anticancer agent or the like is decided for treatment of the cancer.

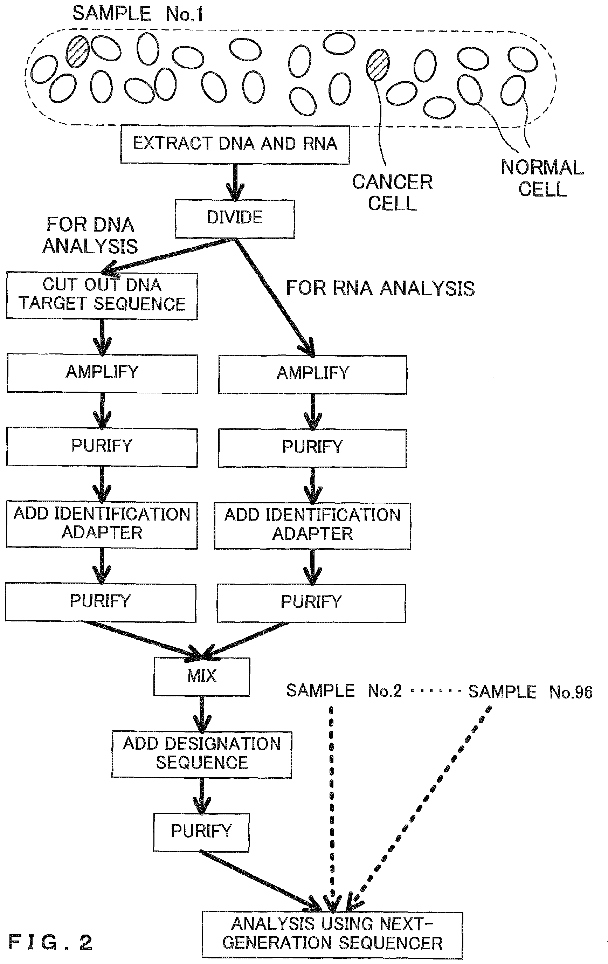

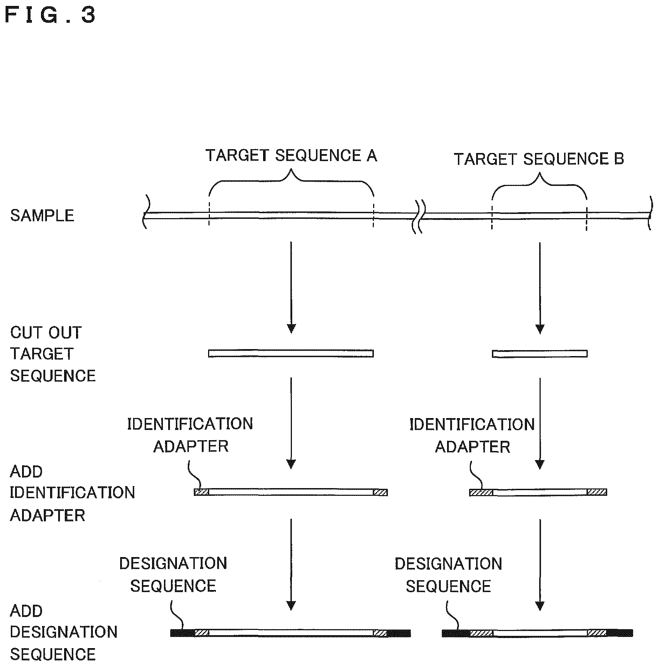

FIG. 2 and FIG. 3 illustrate the outline of methods for genetic testing. A sample No. 1 is a sample obtained from a single patient or a single affected site. The sample contains normal cells and cancer cells. In the case of a sample obtained from a patient with lung cancer by cytologic washings or the like using a bronchoscope, the ratio of the cancer cells to the entire cells is approximately several percent.

From a sample on which genetic testing is to be performed, DNA and RNA are extracted. By using reverse transcriptase, RNA is converted into complementary deoxyribonucleic acid (cDNA). The sample is divided into two such as one for use in a DNA analysis and the other for use in an RNA analysis.

Treatment of the sample for use in the DNA analysis will be described. As illustrated in FIG. 3, a target sequence is cut out of the DNA in the sample. The target sequence is a base sequence of a cancer-associated gene. Returning to FIG. 2, the target sequence is amplified by a polymerase chain reaction (PCR) or the like. For example, 34 cycles of PCR are performed to thereby amplify the target sequence by a factor of approximately seventeen billion. The amplified target sequences are purified by a solid phase reversible immobilization (SPRI) technique or the like.

As illustrated in FIG. 3, to both ends of each of the amplified target sequences, identification adapters for identifying a sample are added. The identification adapter is a base sequence specified for each sample. Returning to FIG. 2, addition of the identification adapter is conducted by six cycles of PCR, for example. The target sequence to which the identification adapters are added is purified.

Treatment of the sample for use in the RNA analysis will be described. For example, 34 cycles of PCR are performed to amplify cDNA of the target sequence. The amplified target sequences are purified. To both ends of each of the amplified target sequences, identification adapters for identifying a sample are added. The addition of the identification adapter is conducted by six cycles of PCR, for example. The target sequence to which the identification adapters are added is purified.

One part used for the DNA analysis and the other part used for the RNA analysis into which a single sample has been divided and on which amplification and purification have been performed are mixed with each other. As illustrated in FIG. 3, to both ends of the target sequence to which the identification adapters have been added, designation sequences are added. The designation sequence is a base sequence necessary for amplification and reading of the base sequence by the next-generation sequencer. Returning to FIG. 2, addition of the designation sequence is conducted by six cycles of PCR, for example. The target sequence to which the designation sequences and the identification adapters are added is purified.

Ninety-six samples for each of which the above-described treatment has been performed are mixed and then analyzed by the next-generation sequencer. The next-generation sequencer simultaneously reads multiple nucleic acid base sequences and records the base sequences in files for each identification adapter.

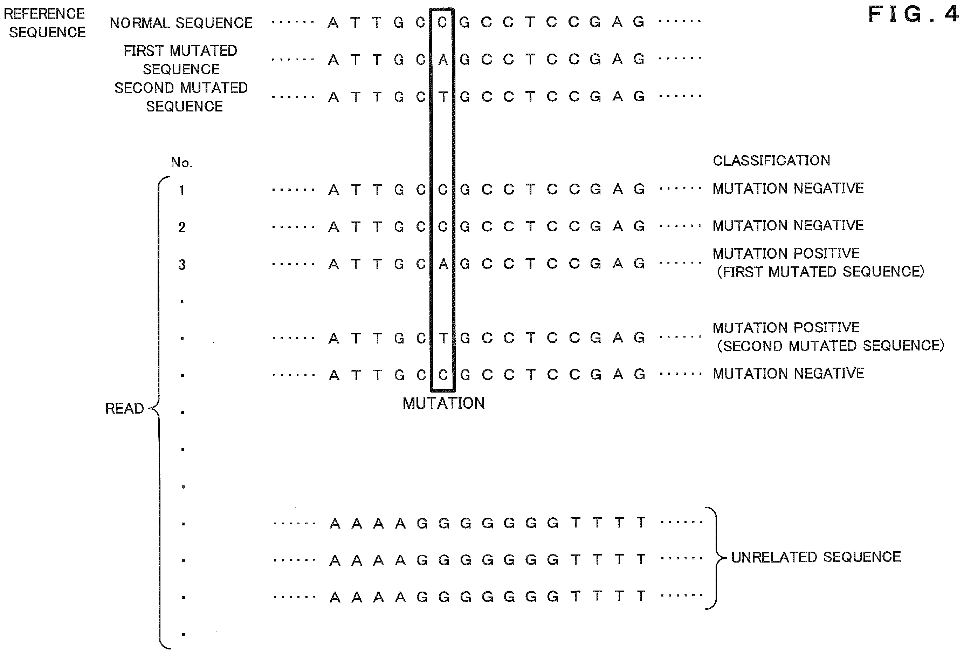

FIG. 4 illustrates one example of reads. A read here means a base sequence read from a single target sequence. The base sequence is represented by four characters of A (adenine), T (timine), G (guanine) and C (cytosine).

A reference sequence includes a normal sequence corresponding to a target sequence and a mutated sequence in which part of the normal sequence is mutated. FIG. 4 illustrates a mutated sequence in which a single base is substituted by another base, though the mutated sequence is not limited to such a single base substitution. The mutated sequence includes a sequence in which a part of the bases of the normal sequence is missing, a sequence in which a base is added at some midpoint of the normal sequence, a sequence in which repetition occurs at a part of the normal sequence, or a sequence having the number of bases different from that of the normal sequence. The mutated sequences associated with a single target sequence are denoted by numbers in an increasing order from a first mutated sequence.

Each read is compared with the reference sequence and classified. The sequence that coincides with the normal sequence is classified as mutation negative. The sequence that coincides with any one of the mutated sequences is classified as mutation positive. The base sequence corresponding to a target sequence different from the reference sequence is classified as an unrelated sequence. By comparison with a reference sequence corresponding to each of the target sequences and classification of the target sequences accordingly, the number of mutation negative sequences, the number of I-th mutation-positive sequences corresponding to the I-th mutated sequence and the number of read sequences n can be determined for each of the target sequences. In the following description, the number of I-th mutation-positive sequences MI may also be referred to as the number of mutation-positive sequences M.

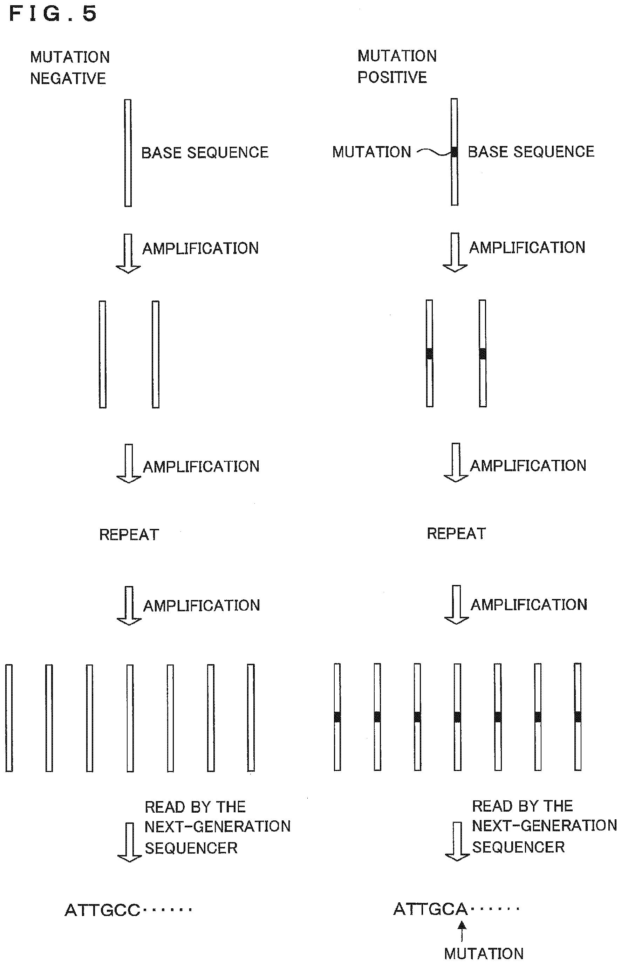

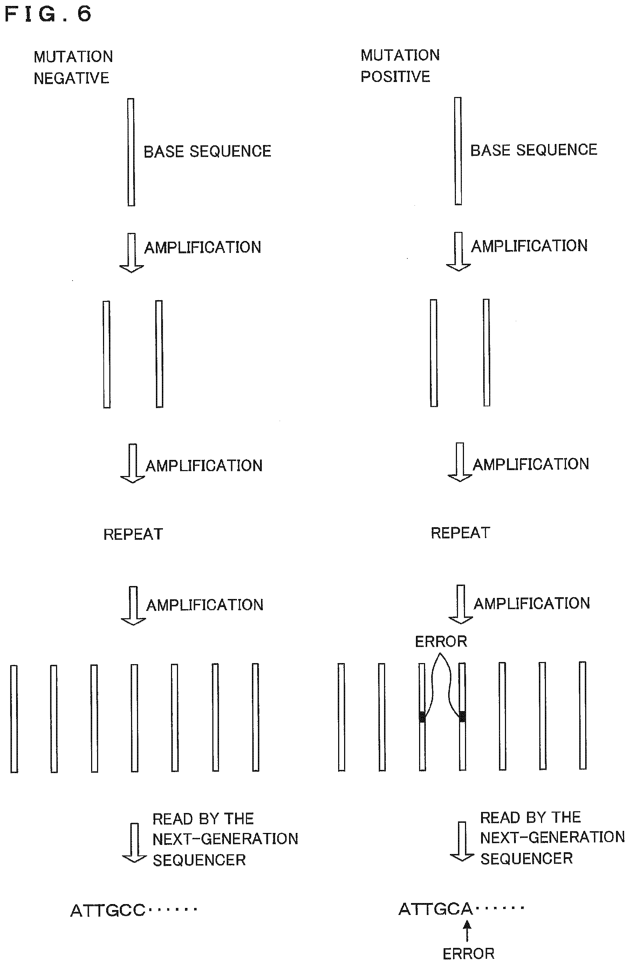

FIG. 5 and FIG. 6 illustrate examples of an amplification procedure of base sequences. The drawing on the left in FIG. 5 shows a case where a base sequence without a mutation is normally amplified. The drawing on the right in FIG. 5 shows a case where a base sequence with a mutation is normally amplified. In either case, the base sequence the same as the original one is copied.

The drawing on the left in FIG. 6 shows a case where a base sequence without a mutation is normally amplified. The base sequence the same as the original one is copied. The drawing on the right in FIG. 6 shows a case where mutations occur due to replication errors during the amplification procedure. Parts of the copied base sequences have sequences different from the original base sequence.

The probability of occurrence of a replication error and a reading error in the next-generation sequencer vary depending on the base sequence of a target sequence, the length of the target sequence, an amplification condition in the pretreatment, the model of the next-generation sequencer and the like. By the preparatory experiment, for each of the target sequences, an error rate p can be investigated that is a probability that a base sequence to be amplified and then read by the next-generation sequencer is different from the original base sequence. The error rate p is the probability of occurrence of a total number of errors obtained by adding the replication errors and the reading errors in the next-generation sequencer.

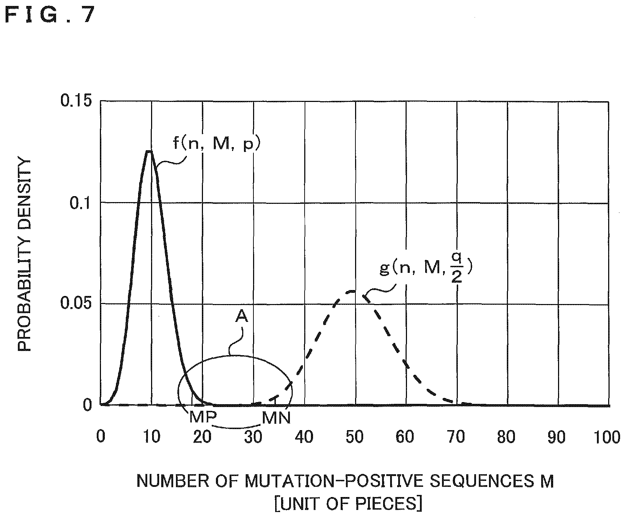

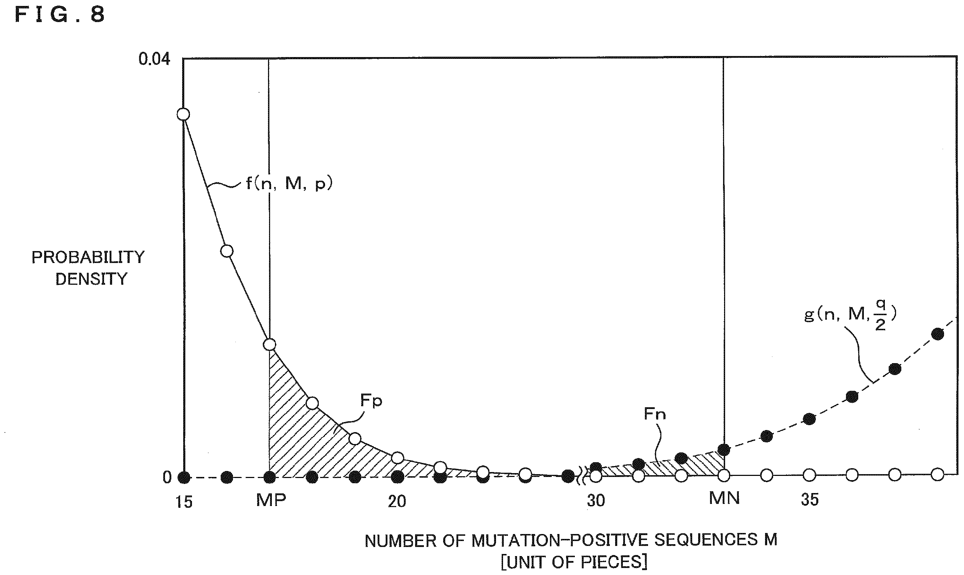

FIG. 7 illustrates a probability density distribution for the number of mutation-positive sequences M associated with a single target sequence. FIG. 8 is an enlarged view of a portion A in FIG. 7. The horizontal axis represents the number of mutation-positive sequences M, which is the number of reads determined to be mutation positive by the unit of pieces. The vertical axis represents a probability density by the unit of a dimensionless quantity. Note that the graphs in FIGs. 7 and 8 are mere examples, and the shape of the graphs change depending on parameters to be described later.

The solid line shows a probability density f (n, M, p) that M pieces of mutation positive reads (also simply referred to as M mutation positive reads) are included in n reads obtained from a sample free of cancer cells. That is, the probability density f is a probability density for M mutation-positive reads as false positives to occur in N reads.

The broken line shows a probability density g (n, M, q/2) for M mutation-positive reads to be included in n reads obtained from a sample containing cancer cells at a detection limit value q. That is, the probability density g is a probability density of M mutation-positive reads as true positives to occur in N reads.

The detection limit value q here means a detection limit value of a ratio of the cancer cells to the entire cells contained in a sample. If the detection limit q is large, a sample being a high percentage of cancer cell content is required to be used. The sample obtained by cytodiognosis is a relatively low percentage of cancer cell content, and thus it is desirable that the detection limit value q is small.

In the following description, the probability density f (n, M, p) and the probability density g (n, M, q/2) may respectively be referred to as the probability density f and the probability density g without describing the variables. The probability density f and the probability density g are functions in which the number of mutation-positive sequences M is 1 if being integrated from 0 to n.

The probability density f is calculated by Equation (1).

[Equation 1]

$$f\left(n, \, M, \, p\right) = {}_{n}C_{M} \, p^{M} \left(1 - p\right)^{(n-M)} \quad \cdots\cdots(1)$$

n is a read sequence number.

m is a mutation-positive sequence number.

p is an error rate which is the sum of replication errors, and reading errors in the next-generation sequencer.

$_{n}C_{m}$ is a binomial coefficient that indicates the number of combinations when selecting M elements from the n element set.

Returning to FIG. 3, if the number of mutation-positive sequences M is equal to or less than a first threshold MP, it is determined that the sample does not contain such mutation associated with the target sequences. Here, if the number of mutation-positive sequences M is larger than the MP, there is a possibility of being determined as false positive. The area denoted by a hatch pattern that slopes downward to the left corresponds to a false positive rate Fp.

The first threshold MP is set to take a maximum number of mutation-positive sequences M when the false positive rate Fp = (1-specificity) is equal to or less than a predetermined value. By adjustment of the first threshold MP, the specificity of the genetic testing can be regulated.

The probability density g is calculated by Equation (2).

[Equation 2]

$$g\left(n, M, \frac{q}{2}\right) = {}_nC_M \left(\frac{q}{2}\right)^M \left(1 - \frac{q}{2}\right)^{(n-M)} \quad \cdots\cdots (2)$$

q is the detection limit value of cancer cells.

If the number of mutation-positive sequences M is larger than a second threshold MN, it is determined that the sample contains some mutations associated with the target sequences.

Here, if the number of mutation-positive sequences M is equal to or less than the MN, there is a possibility of being determined as false negative. The area denoted by a hatch pattern that slopes downward to the right corresponds to a false negative rate Fn.

The second threshold MN is set to take a minimum number of mutation-positive sequences M when the false negative rate Fn = (1-sensitivity) is equal to or less than a predetermined value.

By adjustment of the second threshold MN, the sensitivity of the genetic testing can be regulated.

If the number of read sequences n is small, if the error rate p is large, and if the detection limit value q is small, the MP may be larger than the MN. In such a case, it is impossible to determine whether or not a sample contains mutations associated with the target sequences.

If the detection limit q is set to be large, for example, the MP may be equal to or less than the MN. This corresponds to a situation where the ratio of the cancer cells contained in the sample is low, no mutation in the target sequences is detected, and thus it can be determined as mutation negative.

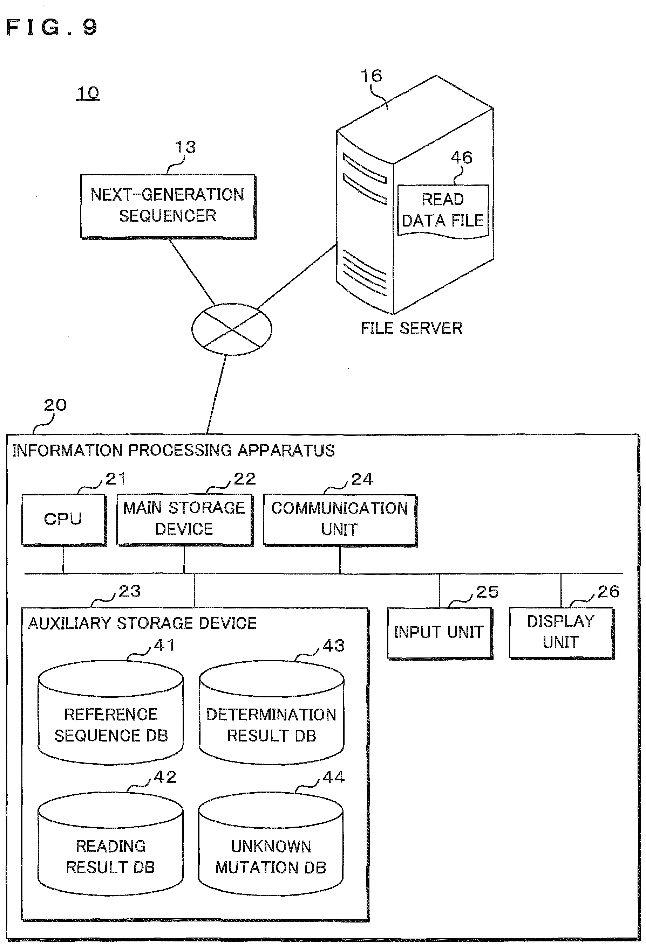

FIG. 9 illustrates the configuration of a genetic testing system 10. The genetic testing system 10 includes a next-generation sequencer 13, a file server 16 and an information processing apparatus 20 that are connected through a network.

The next-generation sequencer 13 reads the base sequences of the samples on which the pretreatment have been performed as described using FIG. 2 and stores a read data file 46 recorded in a predetermined format in the file server 16. The details of the configurations of the next-generation sequencer 13 and the file server 16 are not described here. Noted that the next-generation sequencer 13 and the file server 16 may integrally be configured with each other.

The information processing apparatus 20 includes a central processing unit (CPU) 21, a main storage device 22, an auxiliary storage device 23, a communication unit 24, an input unit 25, a display unit 26 and buses. The CPU 21 is an arithmetic and control unit that executes programs according to the present embodiment. The CPU 21 may employ one or more CPUs or a multi-core CPU, or the like. The CPU 21 is connected to the respective components of the hardware forming the information processing apparatus 20 through the buses.

The main storage device 22 is a storage device such as a static random access memory (SRAM), a dynamic random access memory (DRAM), a flash memory or the like. The main storage device 22 temporarily stores information needed in the middle of the processing performed by the CPU 21 and a program that is being executed by the CPU 21.

The auxiliary storage device 23 is a storage device such as an SRAM, a flash memory, a hard disk or the like. The auxiliary storage device 23 stores a reference sequence DB 41, a reading result DB 42, programs to be executed by the CPU 21 and various data needed to execute programs.

The communication unit 24 is an interface making data communication between the information processing apparatus 20 and the network. The input unit 25 is a keyboard, a mouse and the like. The display unit 26 is a liquid crystal display panel, an organic electro-luminescence (EL) display panel or the like.

The information processing apparatus 20 according to the present embodiment is an information processing apparatus such as a general-purpose personal computer, a tablet or the like. The information processing apparatus 20 may integrally be formed with the next-generation sequencer 13 or the file server 16. The information processing apparatus 20, the next-generation sequencer 13 and the file server 16 may integrally be formed. The information processing apparatus 20 may be a virtual machine operating on one or more large scale computers.

The reference sequence DB 41 and the reading result DB 42 may be stored in a storage device such as the file server 16, a data server connected to the information processing apparatus 20 through the network or the like. The respective DBs may be stored in separate storage devices.

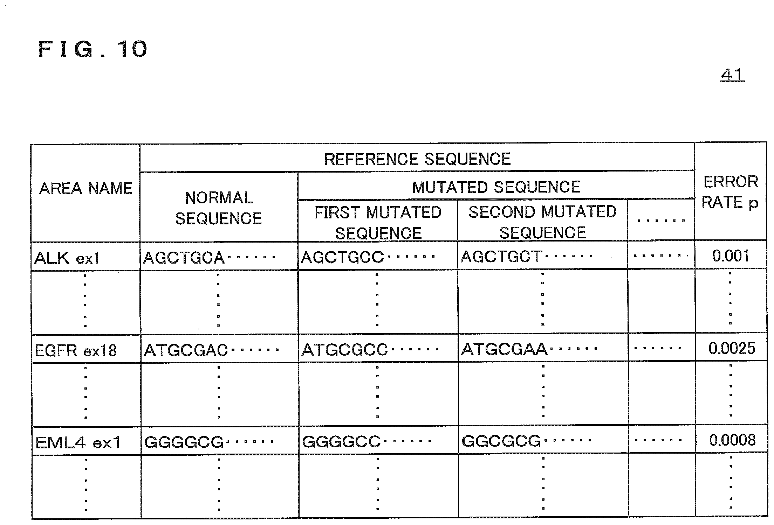

FIG. 10 illustrates a record layout of the reference sequence DB 41. The reference sequence DB 41 is a DB that records an area name, a reference sequence and an error rate p in association with one another. The reference sequence DB 41 includes an area name field, a reference sequence field and an error rate p field. The reference sequence field includes a normal sequence field and a mutated sequence field. The mutated sequence field includes mutated sequence sub-fields denoted by numbers in an increasing order from a first mutated sequence field.

In the area name field, the area name of a target sequence is recorded. In the normal sequence field, a normal base sequence is recorded. In the mutated sequence field, a known mutated sequence is recorded. Here, the number of known mutated sequences is different depending on the target sequence. For the record associated with a target area including a few mutated sequences, the latter part of the mutated sequence field is a blank field. In the error rate p field, an error rate p calculated by a preparatory examination or the like is recorded.

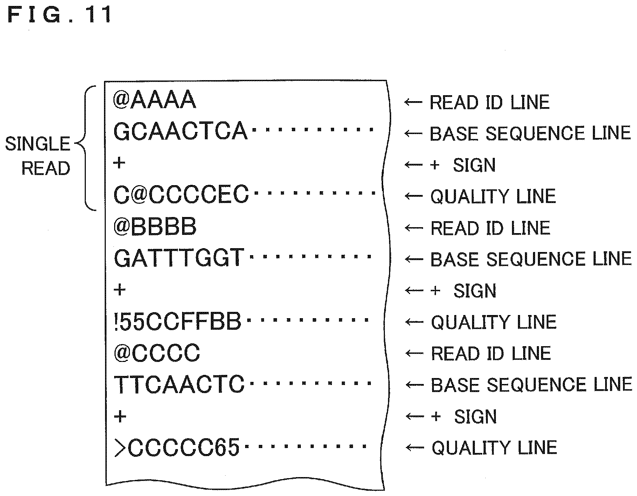

FIG. 11 illustrates one example of the read data file 46. FIG. 11 shows one example of a file in the FASTQ format. In the FASTQ file format, data on a single read is recorded in four lines including a read ID line, a base sequence line, a + (plus) sign and a quality line.

In the read ID line, a read ID uniquely assigned to a read by the next-generation sequencer is recorded. In the base sequence line, a base sequence read by the next-generation sequencer is recorded. In the quality line, a quality value of the bases recorded in the base sequence line, that is, an indicator for reliability is recorded.

As described before, the next-generation sequencer 13 creates the read data file 46 for each identification adapter described by referring to FIG. 3. Hence, reads for a single sample are recorded in one read data file 46. The next-generation sequencer 13 records the result of a single analysis in one folder. Note that the format of the read data file 46 is not limited to the FASTQ format.

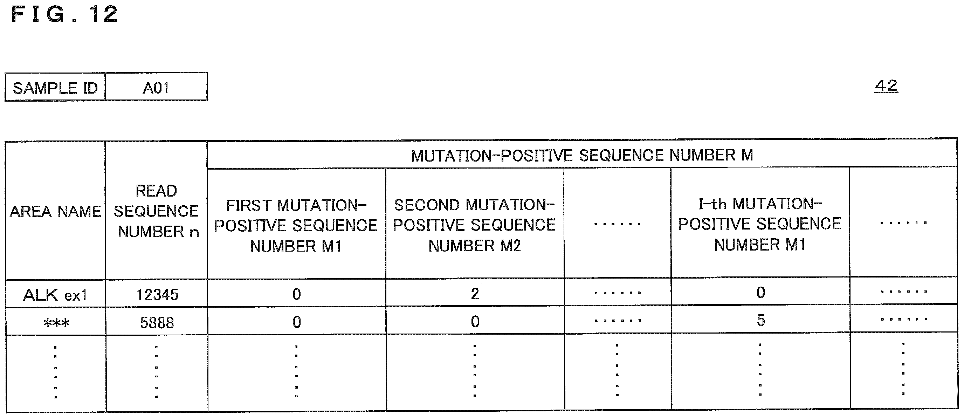

FIG. 12 illustrates a record layout of the reading result DB 42. The reading result DB 42 is a DB that records a sample ID uniquely provided to a sample, an area name and a reading result read by the next-generation sequencer in association with one another.

The reading result DB 42 includes a sample ID field, an area name field, a read sequence number n field and a mutation-positive sequence number M field. The mutation-positive sequence number M field includes the I-th mutation-positive sequence number MI field such as a first mutation-positive sequence number M1 field, a second mutation-positive sequence number M2 field, etc.

In the sample ID field, a sample identifier (ID) uniquely assigned to a sample is recorded. In the area name field, the area name of a target sequence is recorded. In the read sequence number n field, the number of read sequences n of a target sequence is recorded.

In the first mutation-positive sequence number M1 field, the number of mutation-positive reads corresponding to the first mutated sequence field of the reference sequence DB 41 is recorded.

In the second mutation-positive sequence number M2 field, the number of mutation-positive reads corresponding to the second mutated sequence field of the reference sequence DB 41 is recorded.

In the I-th mutation-positive sequence number MI field, the number of mutation-positive reads corresponding to the I-th mutated sequence field of the reference sequence DB 41 is recorded. The reading result DB 42 includes a single record for one area of a single sample.

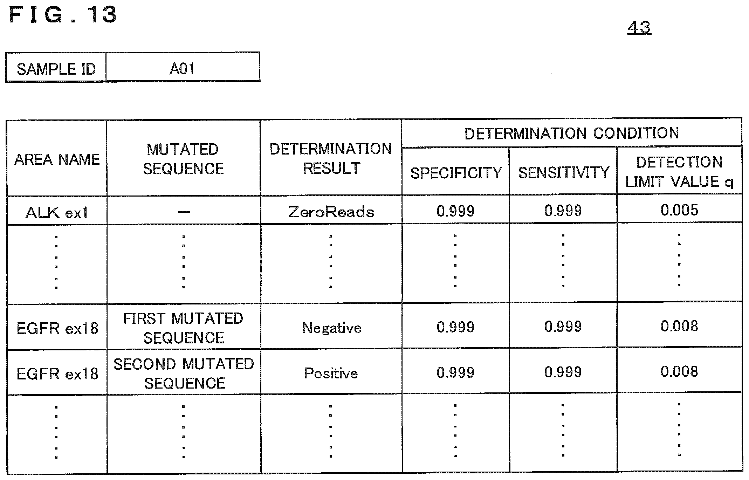

FIG. 13 illustrates a record layout of a determination result DB 43. The determination result DB 43 is a DB that records a sample ID uniquely assigned to a sample, an area name and a determination result in association with one another.

The determination result DB 43 includes a sample ID field, an area name field, a mutated sequence field, a determination result field and a determination condition field. The determination condition field includes a specificity field, a sensitivity field and a detection limit value q field.

In the sample ID field, a sample ID uniquely assigned to a sample is recorded. In the area name field, the area name of a target sequence is recorded. In the mutated sequence field, the name of a mutated sequence is recorded. The "-" in the mutated sequence field means that the type of the mutated sequence is not specified.

In the determination result field, a determination result is recorded. "ZeroReads" indicates that no reads are detected, and "Positive" indicates that the reads are mutation positive while "Negative" indicates that the reads are mutation negative. In the specificity field, specificity used for determination is recorded. In the sensitivity field, sensitivity used for determination is recorded. In the detection limit value q field, a detection limit value q used for determination is recorded.

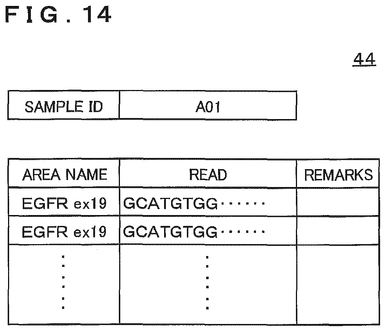

FIG. 14 illustrates a record layout of an unknown mutation DB 44. The unknown mutation DB 44 includes a sample ID field, an area name field, a read field and a remarks field. In the sample ID field, a sample ID uniquely assigned to a sample is recorded. In the area name field, the area name of a target sequence is recorded. In the read field, the base sequence of a read determined to be an unknown mutation is recorded. It is noted that a read ID may be recorded in the read field in place of the base sequence. In the remarks field, remarks are recorded.

The unknown mutation will be described. For example, the EGFR ex19 area and the ALK area include a large number of mutants. The mutants each include a so-called unknown mutation, which has not yet been reported to in medical conferences or the like.

In the tail end of the mutated sequence sub-field of the reference sequence DB 41, a mutated sequence that is a base sequence a part of which is replaced with a so-called wildcard has been stored, which enables detection of a read with an unknown mutation that does not coincide with any known mutated sequence. The base sequence of such a read is recorded in the read field.

This allows the data stored in the unknown mutation DB 44 to be researched and used for a study of a treatment method or the like.

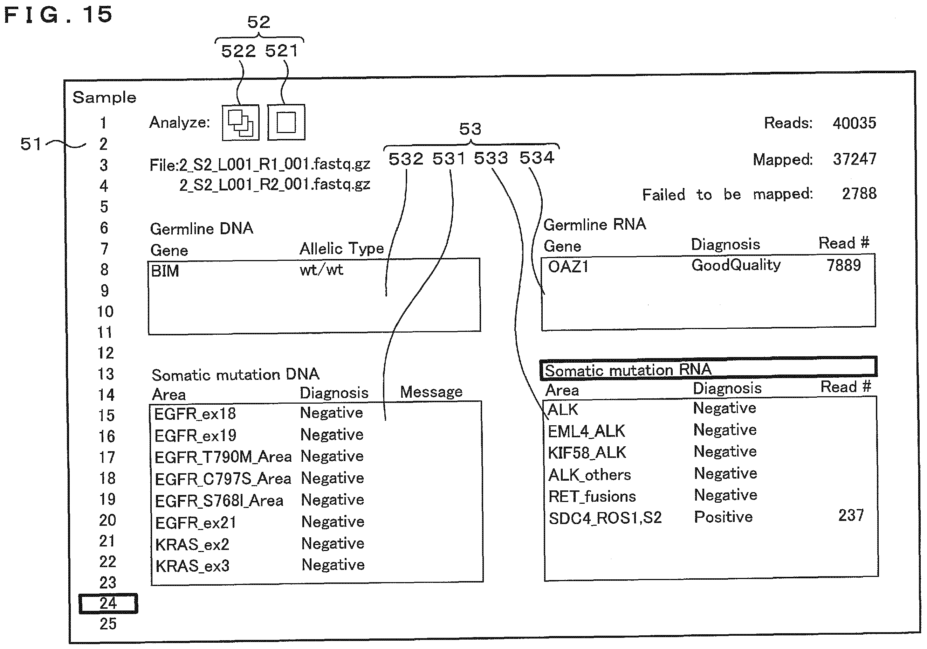

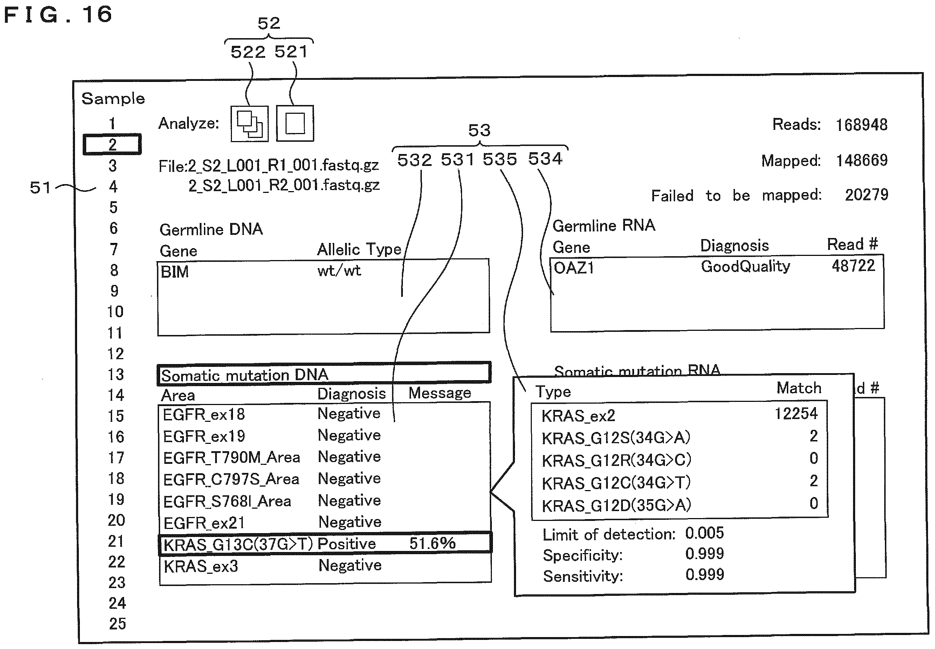

FIG. 15 and FIG. 16 are examples of screens on which determination results are displayed. The screen shown in FIG. 15 includes a sample selection section 51, an analysis button section 52 and an analysis result section 53. The analysis button section 52 includes a single analysis button 521 and a multi-analysis button 522. The analysis result section 53 includes a first analysis result part 531, a second analysis result part 532, a third analysis result part 533 and a fourth analysis result part 534.

If the single analysis button 521 is selected by the user, the CPU 21 obtains a read data file 46 corresponding to one sample designated by the user and analyzes the obtained file. If the multi-analysis button 522 is selected by the user, the CPU 21 obtains multiple read data files 46 stored in a single folder designated by the user and analyzes the obtained files. FIG. 15 shows a screen obtained after the multi-analysis button 522 is selected and analysis has been carried out.

In the first analysis result part 531, an analysis result related to somatic mutation DNA in the target sequences is displayed. In the second analysis result part 532, an analysis result related to germ cell line DNA in the target sequences is displayed. In the third analysis result part 533, an analysis result related to somatic mutation RNA in the target sequences is displayed. In the fourth analysis result part 534, an analysis result related to germ cell line RNA in the target sequences is displayed.

The first analysis result part 531 will be described in more detail. A single line of the first analysis result part 531 shows the analysis result of one target sequence. For example, the first line of the first analysis result part 531 shows that the exon 18 area of the epidermal growth factor receptor (EGFR) gene as one of the oncogenes is determined as mutation negative.

The user can peruse the analysis result of a desired sample by using the sample selection section 51. In FIG. 15, the sample of No.24 is being selected. FIG. 16 shows a state in which the analysis result of the sample of No. 2 different from that in FIG. 15 is being displayed.

In FIG. 16, the first analysis result part 531 shows that KRAS_G13C (37G>T) is mutation positive, and 51.6% of cancer cells are estimated to be contained in the sample. The fifth analysis result part 535 is displayed so as to be popped-up to show the details of the number of associated reads. At the lower part of the fifth analysis result part 535, the detection limit value q, specificity and sensitivity used for the analysis are displayed.

As illustrated in the third analysis result part 533 in FIG. 15 and the first analysis result part 531 in FIG. 16, if any one of the target sequences is mutation positive, the title of the analysis result section 53 is enclosed by a box. This allows the user to easily notice that a target sequence determined to be mutation positive is included. It has been known that, for lung cancer, the effectiveness of treatment with a drug is substantially different depending on the presence or absence of mutations in target sequences. The user can thus appropriately select a drug to be administered to a patient by referring to the analysis results illustrated in FIG. 15 and FIG. 16.

The CPU 21 may display the name of a drug to be recommended based on a target sequence determined as mutation positive. For example, if EGFR ex19 deletion or EGFR L858R mutation in the sample is detected, the CPU 21 displays the names of drugs such as gefitinib, erlotinib, afatinib, etc. The name of a drug may be displayed using a trade name.

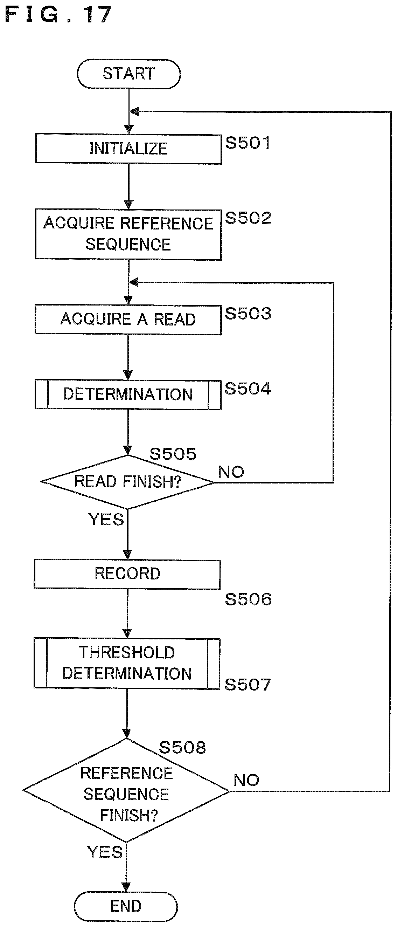

FIG. 17 is a flowchart showing the flow of the processing of a program. The CPU 21 initializes the variables used for counting the number of reads to zero (step S501). The CPU 21 acquires a single reference sequence record from the reference sequence DB 41 (step S502). The CPU 21 acquires the base sequence of a single read from the read data file 46 (step S503).

The CPU 21 activates a subroutine for determination (step S504). The subroutine for the determination is a subroutine for comparing the base sequence of the read acquired at step S503 and the reference sequence acquired at step S502, and determining whether or not the read corresponds to the reference sequence.

The flow of the subroutine for the determination will be described later.

The CPU 21 determines whether or not processing for all the reads recorded in one read data file 46 has been finished (step S505). If determining that the processing has not yet been finished (NO at step S505), the CPU 21 returns to step S503.

If determining that the processing has been finished (YES at step S505), the CPU 21 adds a new record to the reading result DB 42 and records data in the respective fields (step S506). The CPU 21 activates a subroutine for threshold determination (step S507).

The subroutine for the threshold determination is a subroutine for determining a threshold to judge whether or not a target sequence is mutation positive or mutation negative based on predetermined specificity, and sensitivity and a predetermined detection limit value q. The flow of the processing of the subroutine for the threshold determination will be described later.

The CPU 21 determines whether or not processing for all the records in the reference sequence DB 41 has been finished (step S508). If determining that the processing has not yet been finished (NO at step S508), the CPU 21 returns to step S501. If determining that the processing has been finished (YES at step S508), the CPU 21 ends the processing.

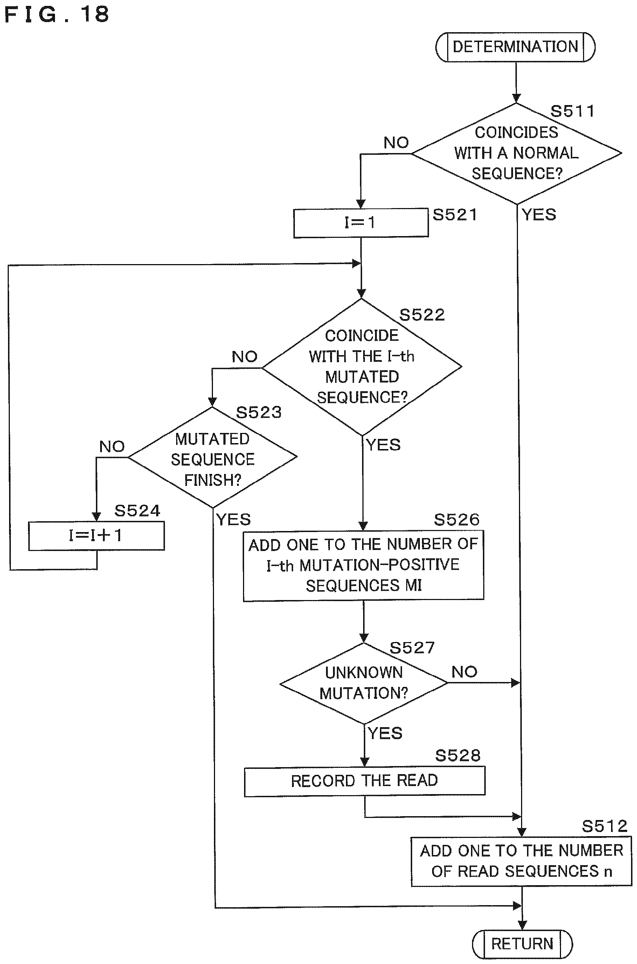

FIG. 18 is a flowchart showing the flow of the processing of the subroutine for the determination. The subroutine for the determination is a subroutine for comparing the base sequence of a read and a reference sequence, and determining whether or not the read corresponds to the reference sequence.

The CPU 21 determines whether or not a read coincides with a normal sequence recorded in the normal sequence field of the reference sequence record (step S511). If determining that the read does not coincide with the normal sequence (NO at step S511), the CPU 21 sets a counter I to 1 (step S521). The CPU 21 determines whether or not the read coincides with the I-th mutated sequence recorded in the I-th mutated sequence field of the reference sequence record (step S522).

If determining that the read does not coincide with the I-th mutated sequence (NO at step S522), the CPU 21 determines whether or not the processing for all the mutated sequences recorded in the reference sequence record that is being processed has been finished (step S523). If determining that the processing has not yet been finished (NO at step S523), the CPU 21 adds one to the counter I (step S524). The CPU 21 returns to step S522. If determining that the processing has been finished (YES at step S523), the CPU 21 ends the processing.

If determining that the read coincides with the I-th mutated sequence (YES at step S522), the CPU 21 adds one to a variable for counting the number of I-th mutation-positive sequences MI (step S526).

The CPU 21 determines whether or not the read that is being processed is an unknown mutation (step S527). If the I-th mutated sequence includes a wildcard as described above, the CPU 21 determines that the read that is being processed is an unknown mutation.

If determining that the read is an unknown mutation (YES at step S527), the CPU 21 creates a record in the unknown mutation DB 44 and records the read therein (step S528). If determining that the read is not an unknown mutation (NO at step S527), if determining that the read coincides with the normal sequence (YES at step S511) or after completion of step S528, the CPU 21 adds one to a variable for counting the number of read sequences n (step S512). The CPU 21 ends the processing.

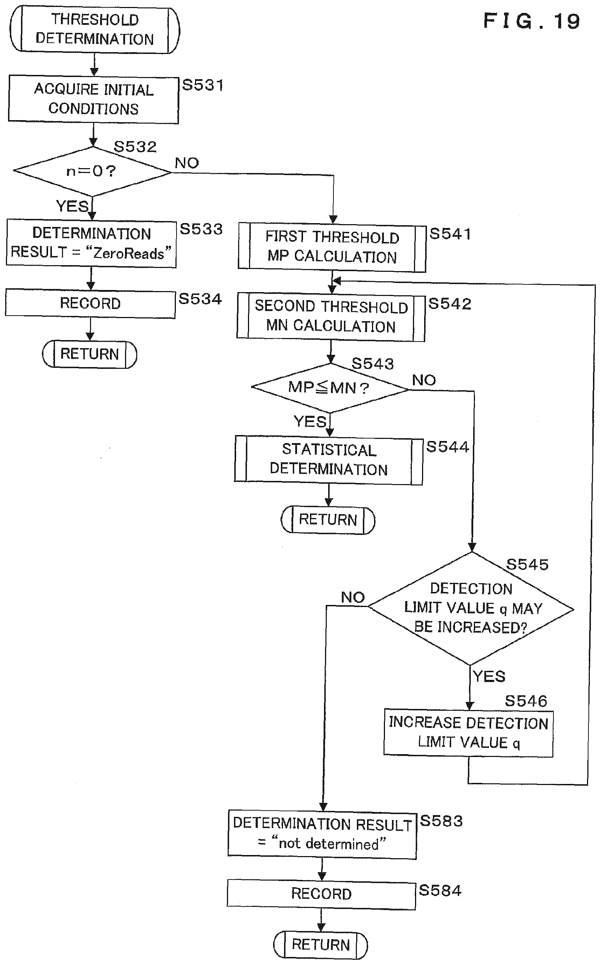

FIG. 19 is a flowchart showing the flow of the processing of the subroutine for the threshold determination. The subroutine for the threshold determination is a subroutine for determining a threshold to judge whether a target sequence is mutation positive or mutation negative based on predetermined specificity, sensitivity and a predetermined detection limit value q.

The CPU 21 acquires initial conditions related to specificity, sensitivity and a detection limit value q (step S531). Note that the initial conditions related to the specificity, the sensitivity and the detection limit value q are stored in the auxiliary storage device 23. The CPU 21 may accept an input of initial conditions by the user.

The CPU 21 determines whether or not the number of read sequences n is zero (step S532). If determining that it is zero (YES at step S532), the CPU 21 determines that the determination result is "ZeroReads", that is, the read corresponding to the reference sequence that is being processed is not included in the read data file 46 (step S533).

The CPU 21 adds a new record to the determination result DB 43. The CPU 21 records the results in the respective fields (step S534). More specifically, the CPU 21 records the sample ID and the area name of the reference sequence that is being processed in the sample ID field and the area name field, respectively. The CPU 21 records "-" meaning that the type of the mutated sequence is not specified in the determination result field. The CPU 21 records "ZeroReads" in the determination result field. The CPU 21 records the specificity, the sensitivity and the detection limit value q in the determination condition field. The CPU 21 ends the processing.

If determining that the number of read sequences n is not zero (NO at step S532), the CPU 21 activates a subroutine for the first threshold MP calculation (step S541). The subroutine for the first threshold MP calculation is a subroutine for calculating the first threshold MP related to specificity as described by referring to FIGs. 7 and 8. The flow of the processing of the subroutine for the first threshold MP calculation will be described later.

The CPU 21 activates a subroutine for the second threshold MN calculation (step S542). The subroutine for the second threshold MN calculation is a subroutine for calculating the second threshold MN related to sensitivity as described by referring to FIGs. 7 and 8. The flow of the processing of the subroutine for the second threshold MN calculation will be described later.

The CPU 21 determines whether or not the first threshold MP is equal to or less than the second threshold MN (step S543). If determining that the first threshold MP is equal to or less than the second threshold MN (YES at step S543), the CPU 21 activates a subroutine for statistical determination (step S544). The subroutine for statistical determination is a subroutine for comparing the number of mutation-positive sequences M recorded in the reading result DB 42 with the second threshold MN and statistically determining the possibility of being mutation positive. The flow of the processing of the subroutine for statistical determination will be described later. Then, the CPU 21 ends the processing.

If determining that the first threshold MP is above the second threshold MN (NO at step S543), the CPU 21 determines whether or not increase in the detection limit value q is allowed (step S545). A maximum value for the detection limit value q is recorded in the auxiliary storage device 23. The CPU 21 may accept an input of a maximum value for the detection limit value q by the user.

If determining that increase is allowed (YES at step S545), the CPU 21 increases the detection limit value q (step S546). An increase at a time is recorded in the auxiliary storage device 23.

The CPU 21 may accept an input of an increase of the detection limit value q by the user, or accept an input of the increased detection limit value q. The CPU 21 returns to step S542.

If determining that increase is not allowed (NO at step S545), the CPU 21 determines that the determination result is "not determined", that is, whether the reference sequence that is being processed is mutation positive or mutation negative cannot be judged (step S547).

The CPU 21 adds a new record to the determination result DB 43. The CPC 21 records the results in the respective fields (step S548). More specifically, the CPU 21 records the sample ID and the area name of the reference sequence that is being processed in the sample ID field and the area name field, respectively. The CPU 21 records "-" meaning that the type of the mutated sequence is not specified in the determination result field. The CPU 21 records "not determined" in the determination result field. The CPU 21 records the specificity, the sensitivity and the detection limit value q in the determination condition field. The CPU 21 ends the processing.

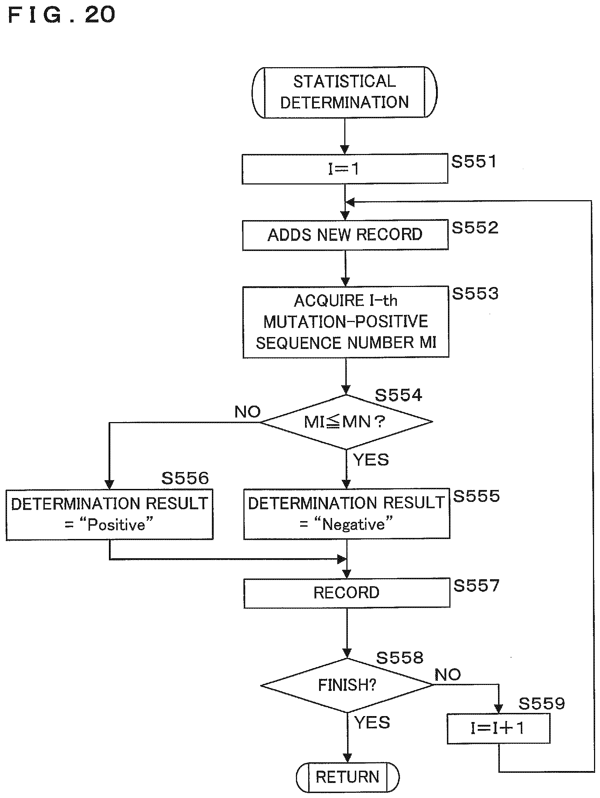

FIG. 20 is a flowchart showing the flow of the processing of the subroutine for statistical determination. The subroutine for statistical determination is a subroutine for comparing the number of mutation-positive sequences M recorded in the reading result DB 42 and the second threshold MN, and statistically determining the possibility of being mutation positive.

The CPU 21 sets the counter I to the initial value 1 (step S551). The CPU 21 adds a new record to the determination result DB 43 (step S552). The CPU 21 records the sample ID and the area name of the reference sequence that is being processed in the sample ID field and the area name field, respectively. The CPU 21 records the specificity, the sensitivity and the detection limit value q in the determination condition field.

The CPU 21 extracts a record from the reading result DB 42 by using the sample ID and the area name as keys. The CPU 21 acquires the number of I-th mutation-positive sequences MI from the I-th mutation-positive sequence number MI field (step S553).

The CPU 21 determines whether or not the number of I-th mutation-positive sequences MI is equal to or less than the second threshold MN (step S554). If determining that the number of I-th mutation-positive sequences MI is equal to or less than the second threshold MN (YES at step S554), the CPU 21 determines that the determination result is "Negative", that is, mutation negative (step S555). If determining that the number of I-th mutation-positive sequences MI is not equal to or less than the second threshold MN (NO at step S554), the CPU 21 determines that the determination result is "Positive", that is, mutation positive (step S556).

After completion of step S555 or S556, the CPU 21 records the determination result obtained at step S555 or step S556 in the determination result field of the record created at step S552 (step S557).

The CPU 21 determines whether or not processing for all the mutated sequence reads have been finished (step S558). If determining that the processing has not yet been finished (NO at step S558), the CPU 21 adds one to the counter I (step S559). The CPU 21 returns to step S552. If determining that the processing has been finished (YES at step S558), the CPU 21 ends the processing.

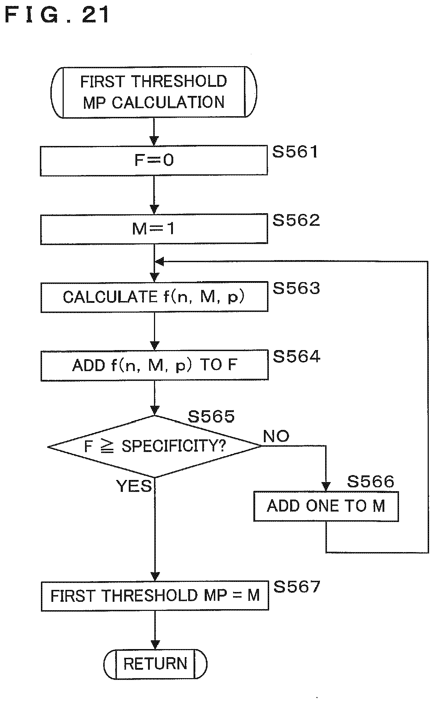

FIG. 21 is a flowchart showing the flow of the processing of the subroutine for the first threshold MP calculation. The subroutine for the first threshold MP calculation is a subroutine for calculating the first threshold MP related to specificity as described by referring to FIGs. 7 and 8.

The CPU 21 sets a variable F to an initial value of 0 (step S561). The CPU 21 sets a variable M to an initial value of 1 (step S562). The CPU 21 calculates f (n, M, p) according to the above-described Equation (1) (step S563), where n means the number of read sequences n obtained from the read sequence number n field of the reading result DB 42, and p is an error rate p obtained from the error rate p field of the reference sequence DB 41.

The CPU 21 adds f (n, M, p) calculated at step S563 to the variable F (step S564). The CPU 21 determines whether or not the variable F is equal to or more than the specificity (step S565). If determining that the variable F is less than the specificity (NO at step S565), the CPU 21 adds one to the variable M (step S566). The CPU 21 returns to step S563.

If determining that the variable F is equal to or more than the specificity (YES at step S565), the CPU 21 determines that the first threshold MP is equal to the variable M (step S567). The CPU 21 ends the processing.

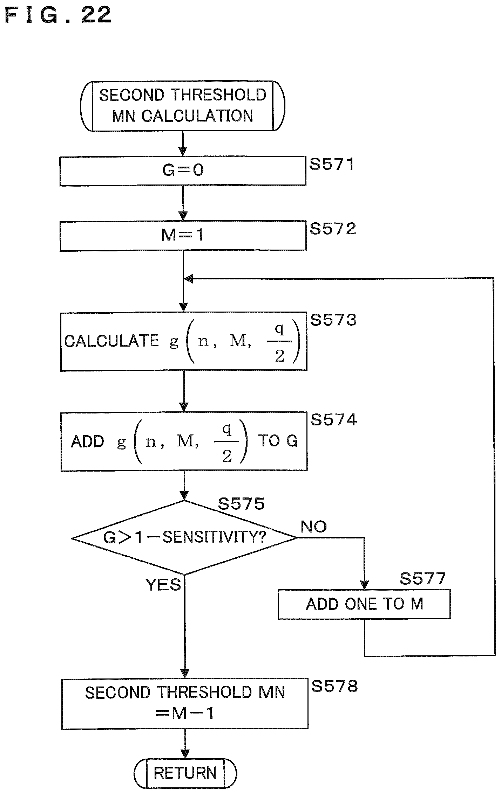

FIG. 22 is a flowchart showing the flow of the subroutine for the second threshold MN calculation. The subroutine for the second threshold MN calculation is a subroutine for calculating the second threshold MN related to sensitivity as described by referring to FIGs. 7 and 8.

The CPU 21 sets a variable G to an initial value of 0 (step S571). The CPU 21 sets the variable M to an initial value of 1 (step S572). The CPU 21 calculates g (n, M, q/2) according to the above-described Equation (2) (step S573), where n means the number of read sequences n obtained from the read sequence number n field of the reading result DB 42, and the detection limit value q is a ratio of the cancer cells to the entire cells contained in the sample.

The CPU 21 adds g (n, M, q/2) calculated at step S573 to the variable G (step S574). The CPU 21 determines whether or not the variable G is above (1-sensitivity) (step S575). If determining that the variable G is not above (1-sensitivity) (NO at step S575), the CPU 21 adds one to the variable M (step S578). The CPU 21 returns to step S573.

If determining that the variable G is above (1-sensitivity) (YES at step S575), the CPU 21 determines that the second threshold MN is equal to (variable M-1) (step S567). The CPU 21 ends the processing.

According to the present embodiment, it is possible to determine whether a base sequence is mutation positive or mutation negative taking into account the probability of occurrence of false positive and false negative due to an error upon amplification of a base sequence. Since the sensitivity, specificity and detection limit value upon determination are clearly shown, the clinician receiving a determination result can select an anticancer agent to be administered taking the reliability of the determination result into account.

According to the present embodiment, even if the number of read sequences n is relatively small, it is possible to determine the possibility of being mutation positive or mutation negative by appropriately adjusting the detection limit value q. Even if a sample is insufficient in quality and quantity, and is hard to recollect, a possible determination is output while the limit is clearly shown, which makes it possible to provide information contributing to the determination by the clinician.

According to the present embodiment, even in the case of a sample being minimal and having a small detection limit value q that is collected by cytodiagnosis or the like using an endoscope, it is possible to determine the possibility of being mutation positive or mutation negative while the sensitivity and the specificity are clearly shown.

According to the present embodiment, the base sequences of multiple samples can simultaneously be read by using a single next-generation sequencer. For example, a clinical examination company that offers an analytical services using a next-generation sequencer can shorten the period from reception of a sample to reporting of the presence or absence of genetic mutations to a medical institution by making effectively use of the performance of the next-generation sequencer. Furthermore, the cost of treating each of the samples can be lowered.

According to the present embodiment, it is possible to determine the probability of being mutation positive or mutation negative for each sample and for each target sequence at predetermined sensitivity and specificity and a predetermined detection limit value q. Accordingly, even if samples of low quality are included in the multiple samples to be simultaneously read, they never affect the determination for the rest of the samples. The samples of low quality here mean samples from which DNA and RNA in sufficient quality and quantity cannot be extracted, that is, samples containing more necrotic tissues, samples that are not appropriately treated after being collected or the like.

According to the present embodiment, the purification process of the base sequence is performed after the amplification process as described by referring to FIG. 2. Primer dimer occurring in the samples of low quality is removed by the purification process, which enables stable reading of the base sequences by the next-generation sequencer.

According to the present embodiment, for target sequences including many mutants such as the EGFR ex19 and the ALK, an unknown mutated sequence that has not yet been recorded in the database can be detected. The detected unknown mutated sequence is recorded in the unknown mutation DB 44, which can contribute to medical research aiming at development of a new method of treatment or the like.

According to the present embodiment, if an important mutated sequence that exerts an influence on selection of an anticancer agent is newly discovered, this mutated sequence is added to the reference sequence DB 41, and the read data file 46 is analyzed again, whereby determination as to this mutated sequence can be performed.

The subject of being tested is not limited to lung cancer.
The genetic testing system 10 can be used for testing a genetic mutation in a sample collected from various sites of a digestive organ, the urinary organs, the genital organs or the like. In this case, oncogenes expressing in the respective organs are recorded in the reference sequence DB 41. Moreover, in the pretreatment, the corresponding oncogenes are cut out and amplified.

It is noted that in the present embodiment, the first threshold MP and the second threshold MN are calculated by using the number of mutation-positive sequences M in Equations (1) and (2) as variables while thresholds corresponding to predetermined sensitivity and specificity may be calculated by using the number of read sequences n as a variable. In such a case, the number of read sequences n actually read by the next-generation sequencer and the calculated thresholds are compared with each other to thereby make determination as to the probability of being mutation positive or mutation negative.

[Embodiment 2]

The present embodiment relates to a genetic testing system 10 that performs determination by dynamically changing a detection limit value q, sensitivity and specificity. The parts common to those in Embodiment 1 are not described here.

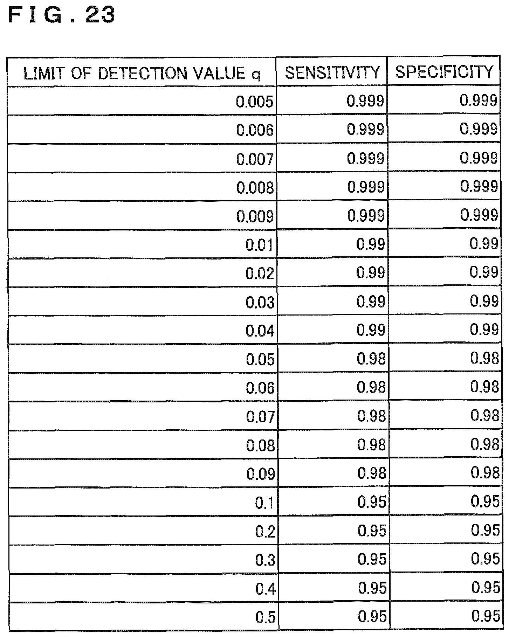

FIG. 23 illustrates a record layout of a parameter DB.
The parameter DB is a DB for recording combinations of the limit of detection value q, sensitivity and specificity. The parameter DB includes a detection limit value q field, a sensitivity field and a specificity field. A detection limit value q, sensitivity and specificity are recorded in the detection limit value q field, the sensitivity field and the specificity field, respectively. In one record of the parameter DB, a combination of a detection limit value q, sensitivity and specificity is recorded.

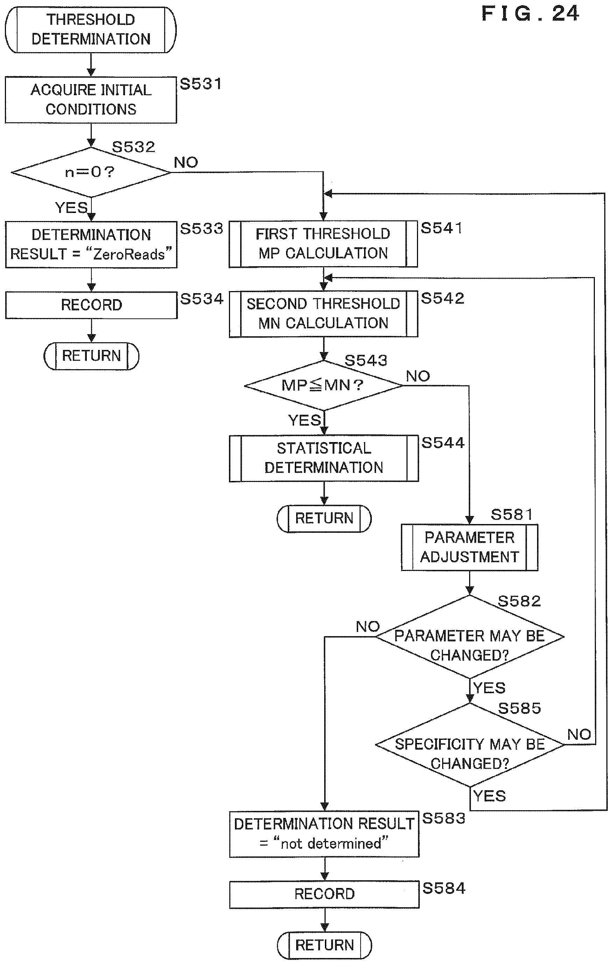

FIG. 24 is a flowchart showing the flow of processing of a subroutine for threshold determination according to Embodiment 2. The subroutine illustrated in FIG. 24 is used in place of the subroutine described by referring to FIG. 19. The processing before step S543 is the same as that described by referring to FIG. 19, and thus the description thereof is not made here.

If determining that the first threshold MP is above the second threshold MN (NO at step S543), the CPU 21 activates a subroutine for parameter adjustment (step S581). The subroutine for parameter adjustment is a subroutine for adjusting three parameters such as the detection limit value q, sensitivity and specificity. The flow of the processing of the subroutine for the parameter adjustment will be described later.

The CPU 21 determines whether or not any one of the parameters or multiple parameters are changed by the subroutine for the parameter adjustment (step S582). If determining that no parameter is changed (NO at step S582), the CPU 21 determines that the determination result is "not determined", that is, that whether the reference sequence that is being processed is mutation positive or mutation negative cannot be judged (step S583).

The CPU 21 adds a new record to the determination result DB 43. The CPU 21 records the results to the respective fields (step S584). More specifically, the CPU 21 records the sample ID and the area name of the reference sequence that is being processed in the sample ID field and the area name field, respectively. The CPU 21 records "-" meaning that the type of the mutated sequence is not specified in the determination result field. The CPU 21 records "not determined" in the determination result field. The CPU 21 records the specificity, the sensitivity and the detection limit value q in the determination condition field. The CPU 21 ends the processing.

If determining that any parameter is changed (YES at step S582), the CPU 21 determines whether or not the specificity is changed (step S585). If determining that the specificity is changed (YES at step S585), the CPU 21 returns to step S541. If determining that the specificity is not changed (NO at step S585), the CPU 21 returns to step S542.

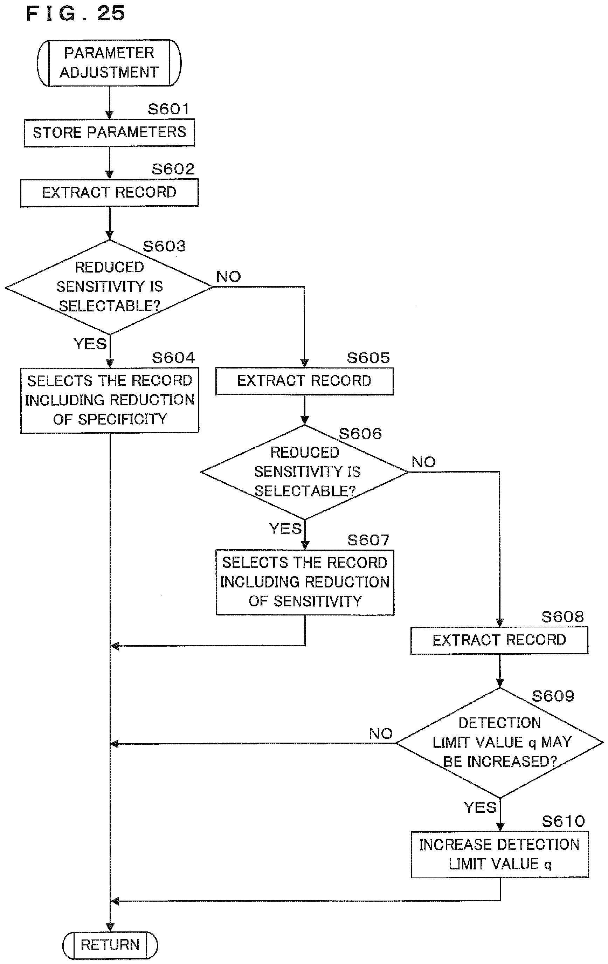

FIG. 25 is a flowchart showing the flow of processing of a subroutine for the parameter adjustment. The CPU 21 temporarily stores three parameters of the detection limit value q, sensitivity and specificity in the auxiliary storage device 23 (step S601).

The CPU 21 extracts all the corresponding records from the parameter DB using the sensitivity and detection limit value q stored at step S601 as keys (step S602). The CPU 21 determines whether or not the record including reduced specificity is selectable from the extracted records (step S603).

If determining that it is selectable (YES at step S603), the CPU 21 selects the record including the least reduction of specificity, and sets the specificity recorded in the specificity field of this record to new specificity (step S604). Then, the CPU 21 ends the processing.

If determining that it is not selectable (NO at step S603), the CPU 21 extracts all the corresponding records from the parameter DB using the specificity and detection limit value q stored at step S601 as keys (step S605). The CPU 21 determines whether or not the record including reduced sensitivity is selectable from the extracted records (step S606).

If determining that it is selectable (YES at step S606), the CPU 21 selects the record including the least reduction of sensitivity, and sets the sensitivity recorded in the sensitivity field of this record to new sensitivity (step S607). Then, the CPU 21 ends the processing.

If determining that it is not selectable (NO at step S606), the CPU 21 extracts all the corresponding records from the parameter DB using the specificity and sensitivity stored at step S601 as keys (step S608). The CPU 21 determines whether or not the record including an increased detection limit value q is selectable (step S609).

If determining that it is selectable (YES at step S609), the CPU 21 selects the record including the least increase of the detection limit value q, and sets the detection limit value q recorded in the detection limit value q field of this record to a new detection limit value q (step S610). Then, the CPU 21 ends the processing. If determining that it is not selectable (NO at step S609), the CPU 21 ends the processing.

According to the present embodiment, it is possible to provide a genetic testing system 10 that performs determination by dynamically changing the sensitivity and specificity in addition to the detection limit value q.

One of the three parameters including the detection limit value q, sensitivity and specificity is fixed while the remaining two parameters may be changed. Two of the three parameters of the detection limit value q, sensitivity and specificity are fixed while the remaining one parameter may be changed.

[Embodiment 3]

The present embodiment relates to a genetic testing system 10 that outputs an estimation result if an undetectable target sequence is present. The parts common to those in Embodiment 1 are not described here.

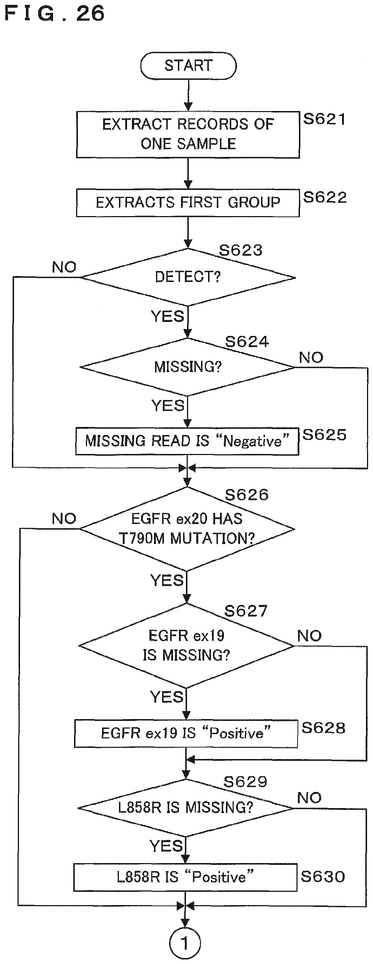

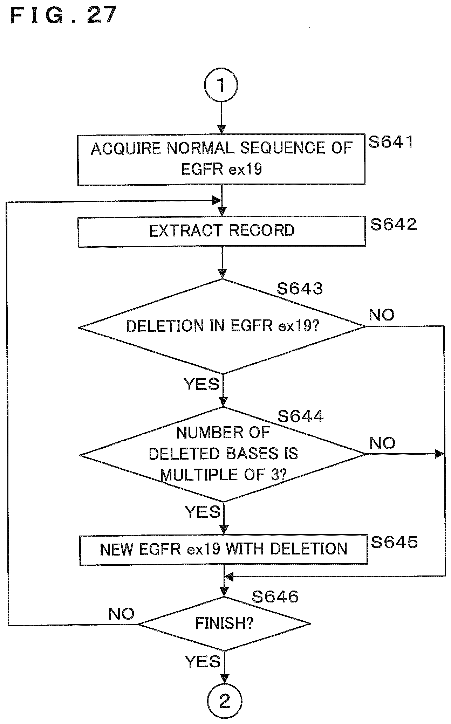

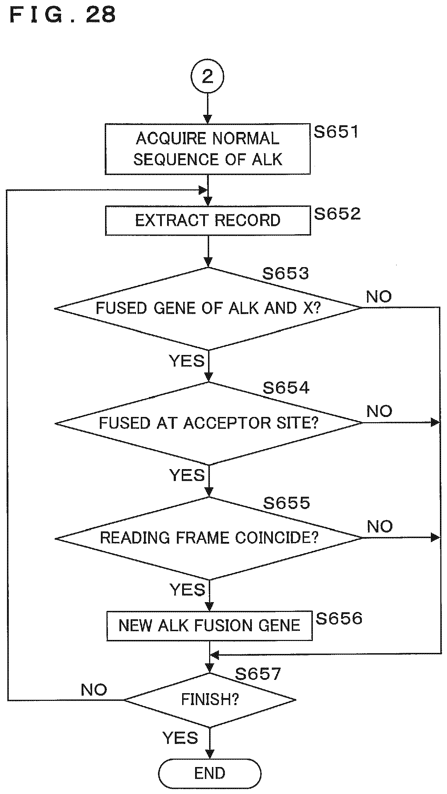

FIGs. 26 to 28 are flowcharts showing the flow of processing of a program according to Embodiment 3. The program from FIGs. 26 to 28 is a program to be executed after the determination results for the respective target sequences are recorded in the determination result DB 43 by the program according to Embodiment 1 or 2.

The CPU 21 extracts records of one sample from the determination result DB 43 using the sample ID as a key (step S621). The CPU 21 further extracts records using the area names related to the genes of the first group shown in Table 1 as keys from the records extracted at step S621 (step S622).

[Table 1]

| Gene name |
| --- |
| KRAS |
| EGFR |
| BRAF |
| ALK fusion gene |
| RET fusion gene |
| ROS1 fusion gene |

The CPU 21 determines whether or not a record for which a read is detected is present among the records extracted at step S622 (step S623). More specifically, it is determined whether or not a record including a determination result other than "ZeroReads" recorded in the determination result field is present.

If determining that a record for which a read is detected is present (YES at step S623), the CPU 21 determines whether or not a record for which a part of reads is missing is present among the records extracted at step S622. More specifically, it is determined whether or not a record including "ZeroReads" recorded in the determination result field is present (step S624). If determining that a record for which a part of reads is missing is present (YES at step S624), the CPU 21 changes the determination result field of this record to "Negative" (step S625).

If the determination result field is to be changed, the CPU 21 may change the respective sub-fields in the determination condition field for the same record to "-" to thereby show that this is an estimation based on the determination result of another record. The CPU 21 may record that this is an estimation based on the determination result of another record in the remarks field of the determination result DB 43.

If determining that the a record for which a read is detected is not present (NO at step S623), if determining that a record for which a read is missing is not present (NO at step S624), or after completion of step S625, the CPU 21 extracts records related to T790M mutation in the EGFR ex20 from the records extracted at step S621. The CPU 21 determines whether or not "Positive" is recorded in the determination result field of the extracted records (step S626).

If determining that "Positive" is recorded (YES at step S626), the CPU 21 extracts records related to the EGFR ex19 from the records extracted at step S621. The CPU 21 determines whether or not "ZeroReads" is recorded in the determination result field of the extracted records (step S627).

If determining that "ZeroReads" is recorded (YES at step S627), the CPU 21 changes the determination result field of the records related to the EGFR ex19 to "Positive" (step S628).

If determining that "ZeroReads" is not recorded (NO at step S627), or after completion of step S628, the CPU 21 extracts records related to the EGFR L858R mutation from the records extracted at step S621. The CPU 21 determines whether or not "ZeroReads" is recorded in the determination result field of the extracted records (step S629).

If determining that "ZeroReads" is recorded (YES at step S629), the CPU 21 changes the determination result field of the records related to the EGFR L858R mutation to "Positive" (step S630).

If determining that "Positive" is not recorded in the determination result field of the records related to T790M mutation in the EGFR ex20 (NO at step S626), if determining that "ZeroReads" is not recorded in the determination result field of the records related to the EGFR L858R mutation (NO at step S629), or after the completion of step S630, the CPU 21 extracts records from the reference sequence DB 41 using the area name related to the EGFR ex19 as a key, and acquires the normal sequence of the EGFR ex19 from the normal sequence field (step S641).

The CPU 21 extracts one record from the unknown mutation DB 44 using the area name related to the EGFR ex19 as a key (step S642). The CPU 21 compares the read recorded in the read field of the extracted record and the normal sequence acquired at step S641, and determines whether or not deletion of bases is present in the read (step S643). If determining that deletion of bases is present (YES at step S643), the CPU 21 determines whether or not the number of deleted bases is a multiple of 3 (step S644).

If determining that the number of deleted bases is a multiple of 3 (YES at step S644), the CPU 21 records in the remarks field of the record extracted at step S642 that a new read of the EGFT ex19 including deletion of bases is recorded (step S645).

If determining that deletion of bases is not present (NO at step S643), determining that the number of deleted bases is not a multiple of 3 (NO at step S644), or after completion of step S645, the CPU 21 determines whether or not the processing of the record related to the EGFR ex19 recorded in the unknown mutation DB 44 has been finished (step S646). If determining that it is not yet finished (NO at step S646), the CPU returns to step S642.

If determining that it is finished (YES at step S646), the CPU 21 extracts records from the reference sequence DB 41 using the area name related to the ALK gene as a key, and acquires a normal sequence of the ALK gene from the normal sequence field (step S651). It is noted that the CPU 21 acquires the entire normal sequence of the areas included in the ALK gene.

The CPU 21 extracts one record from the unknown mutation DB 44 using the area name related to the ALK gene as a key (step S652). The CPU 21 compares the read recorded in the read field of the extracted record and the normal sequence acquired at step S651 to determine whether or not the read is a fused gene between the ALK gene and another gene X (step S653).

If determining that the read is a fused gene (YES at step S653), the CPU 21 determines whether or not the fused site between the ALK gene and the gene X is an acceptor site of the ALK ex20 (step S654). If determining that the fused site is the acceptor site of the ALK ex20 (YES at step S654), the CPU 21 determines whether or not a base reading frame of the gene X coincides with a base reading frame of the ALK gene (step S655).

If determining that they coincide with each other (YES at step S655), the CPU 21 records in the remarks field of the record extracted at step S652 that a read of new ALK fusion gene is recorded in this record (step S656).

If determining that the read is not a fused gene (NO at step S653), if determining that the fused site is not the acceptor site of the ALK ex20 (NO at step S654), if determining that they do not coincide with each other (NO step S655), or after completion of step S656, the CPU 21 determines whether or not the processing of the record related to the ALK gene recorded in the unknown mutation DB 44 has been finished (step S657). If determining that the processing has not yet been finished (NO at step S657), the CPU 21 returns to step S652. If determining that the processing has been finished (YEs at step S657), the CPU 21 ends the processing.

The program according to the present embodiment may automatically be executed after the determination results for the respective target sequences are recorded in the determination result DB 43 by the program according to Embodiment 1 or Embodiment 2, or may be executed after reception of an instruction from the user. The program according to the present embodiment may be executed when a clinician, a researcher or the like, who has received analysis results from a clinical examination facility, browses data related to samples.

The program according to the present embodiment may be separated into a part of interpolation of deletion of read from steps S621 to S630 and a part of estimation of a new mutation after step S641, for example, and may be executed according to the selection of either part by the user.

According to present embodiment, it is possible to achieve the genetic testing system 10 that outputs an estimation result if an undetectable target sequence is present. According to present embodiment, it is possible to provide the genetic testing system 10 that detects a new EGFR ex19 deletion gene and a new ALK fusion gene. The information on these new genes is information useful for research of anticancer agents.

[Embodiment 4]

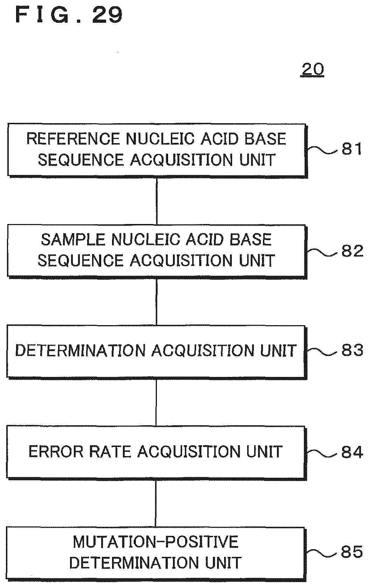

FIG. 29 is a functional block diagram of an information processing apparatus 20. The information processing apparatus 20 includes a reference nucleic acid base sequence acquisition unit 81, a sample nucleic acid base sequence acquisition unit 82, a determination acquisition unit 83, an error rate acquisition unit 84 and a mutation-positive determination unit 85.

The reference nucleic acid base sequence acquisition unit 81 acquires a reference nucleic acid base sequence including a normal sequence and a mutated sequence. The sample nucleic acid base sequence acquisition unit 82 acquires multiple sample nucleic acid base sequences read from a sample. The determination acquisition unit 83 determines the number of read sequences corresponding to the reference nucleic acid base sequence acquired by the reference nucleic acid base sequence acquisition unit 81 and the number of mutated sequences corresponding to the mutated sequence acquired by the reference nucleic acid base sequence acquisition unit 81 among the sample nucleic acid base sequences acquired by the sample nucleic acid base sequence acquisition unit 82.

An error rate acquisition unit 84 acquires an error rate p when the nucleic acid base sequence is read. A mutation-positive determination unit 85 determines whether or not the sample nucleic acid base sequence is mutation positive based on the number of mutated sequences and the number of read sequences that are determined by the determination acquisition unit 83, the error rate p acquired by the error rate acquisition unit 84 and a predetermined false positive rate.

[Embodiment 5]

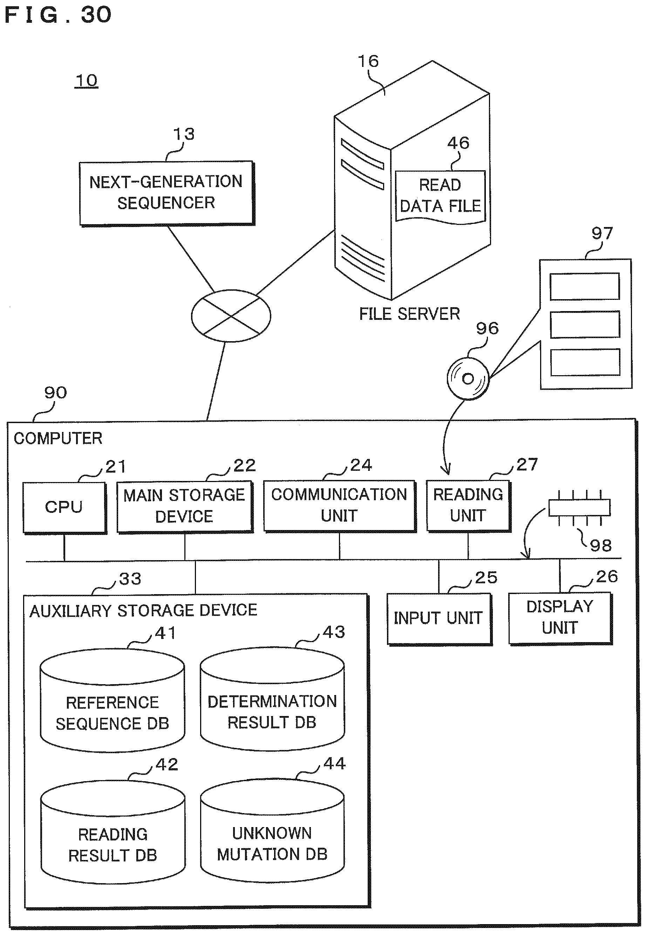

The present embodiment relates to a genetic testing system 10 implemented by operating a general-purpose computer and a program 97 in combination. FIG. 30 illustrates the configuration of the genetic testing system 10 according to Embodiment 5. The parts common to those in Embodiment 1 are not described here.

The genetic testing system 10 according to the present embodiment includes a next-generation sequencer 13, a file server 16 and a computer 90 that are connected through a network. The computer 90 includes a CPU 21, a main storage device 22, an auxiliary storage device 23, a communication unit 24, an input unit 25, a display unit 26, a reading unit 27 and a bus.

The program 95 is recorded into a portable recording medium 96. The CPU 21 reads the program 97 via the reading unit 27 and stores it in the auxiliary storage device 23. The CPU 21 may read out the program 97 stored in a semiconductor memory 98 such as a flash memory or the like mounted on the computer 90. The CPU 21 may download the program 97 from another server computer (not illustrated) connected via the communication unit 24 and the network (not illustrated) and store it in the auxiliary storage device 23.

The program 97 is installed as a control program of the computer 90, and loaded in the main storage device 22 and executed. This allows the computer 90 to function as the above-described information processing apparatus 20.

Any of the technical features (the constituent features) described in the respective embodiments can be combined with each other, and such a combination can form a new technical feature.

It is to be understood that the embodiments disclosed here are illustrative in all respects and not restrictive. The scope of the present invention is defined by the appended claims, not by the above-mentioned meaning, and all changes that fall within the meanings and the bounds of the claims, or equivalence of such meanings and bounds are intended to be embraced by the claims.

[Description of Reference Numerals]

| | |
|---|---|
| 10 | genetic testing system |
| 13 | next-generation sequencer |
| 16 | file server |
| 20 | information processing apparatus |
| 21 | CPU |
| 22 | main storage device |
| 23 | auxiliary storage device |
| 24 | communication unit |
| 25 | input unit |
| 26 | display unit |
| 27 | reading unit |
| 41 | reference sequence DB |
| 42 | reading result DB |
| 43 | determination result DB |
| 44 | unknown mutation DB |
| 46 | read data file |
| 51 | sample selection section |
| 52 | analysis button section |
| 521 | single analysis button |
| 522 | multi-analysis button |
| 53 | analysis result section |
| 531 | first analysis result part |
| 532 | second analysis result part |
| 533 | third analysis result part |
| 534 | fourth analysis result part |

535    fifth analysis result part
81     reference nucleic acid base sequence acquisition unit
82     sample nucleic acid base sequence acquisition unit
83     determination acquisition unit
84     error rate acquisition unit
85     mutation-positive determination unit
90     computer
96     portable recording medium
97     program
98     semiconductor memory


**Claims**

1. A program causing a computer to execute the processing of:

   acquiring a reference nucleic acid base sequence including a normal sequence and a mutated sequence in which a part of the normal sequence is mutated;
   acquiring a plurality of sample nucleic acid base sequences read from a sample;
   determining the number of read sequences corresponding to the reference nucleic acid base sequence and the number of mutated sequences corresponding to the mutated sequence among the sample nucleic acid base sequences;
   acquiring an error rate obtained when a nucleic acid base sequence is read; and
   determining whether or not the sample nucleic acid base sequence is mutation positive based on the number of mutated sequences, the number of read sequences, the error rate and a predetermined false positive rate.

2. The program according to claim 1, wherein whether or not the sample nucleic acid base sequence is mutation positive is determined based on the number of mutated sequences, the number of read sequences, a predetermined false negative rate and a predetermined detection limit value.

3. The program according to claim 2, wherein
   a first threshold is calculated for determining whether or not the sample nucleic acid base sequence is mutation positive based on the number of read sequences, the error rate and a predetermined false positive rate,
   a second threshold is calculated for determining whether or not the sample nucleic acid base sequence is mutation positive based on the number of read sequences, a predetermined false negative rate and a predetermined detection limit value, and
   the sample is determined to be mutation-positive in a case where the second threshold is equal to or larger than the first threshold, and the number of mutated sequences is equal to or larger than the second threshold.

4. The program according to claim 3, wherein the first threshold is determined based on Equation (1) while the second threshold is determined based on Equation (2).
   [Equation 1]

$$1 - \sum_{M=1}^{MP-1} {}_{n}C_{M}\, p^{M}(1-p)^{(n-M)} \leqq Fp$$

$$\leqq 1 - \sum_{M=1}^{M1} {}_{n}C_{M}\, p^{M}(1-p)^{(n-M)} \quad \cdots\cdots \ (1)$$

$$\sum_{M=1}^{MN} {}_n C_M \left(\frac{q}{2}\right)^M \left(1 - \frac{q}{2}\right)^{(n-M)} \leqq F_n$$

$$\leqq \sum_{M=1}^{MN+1} {}_n C_M \left(\frac{q}{2}\right)^M \left(1 - \frac{q}{2}\right)^{(n-M)} \quad \cdots \cdots (2)$$

MP is the first threshold.
MN is the second threshold.
n is a read sequence number.
p is an error rate which is the sum of replication errors, and reading errors in the next-generation sequencer.
q is the detection limit value of cancer cells.
Fp is a false positive rate.
Fn is a false negative rate.

5. The program according to any one of claims 1 to 4, wherein the sample nucleic acid base sequences are read by a next-generation sequencer.

6. The program according to any one of claims 1 to 5, wherein a drug name is output based on the sample nucleic acid base sequences in a case where the sample nucleic acid base sequence is determined to be mutation positive.

7. The program according to any one of claims 1 to 6, wherein
for each of a plurality of reference nucleic acid base sequences, the number of read sequences is acquired, and whether the sample nucleic acid base sequence is mutation positive or not is determined, if the number of read sequences corresponding to a first reference nucleic acid base sequence is equal to or less than a predetermined value, based on the number of read sequences corresponding to another reference nucleic acid base sequence.

8. An information processing method causing a computer to execute the processing of:

acquiring a reference nucleic acid base sequence including a normal sequence and a mutated sequence;
acquiring a plurality of sample nucleic acid base sequences read from a sample;
determining the number of read sequences corresponding to the reference nucleic acid base sequence and the number of mutated sequences corresponding to the mutated sequence among the sample nucleic acid base sequences;
acquiring an error rate when a nucleic acid base sequence is read; and
determining whether or not the sample nucleic acid base sequence is mutation positive based on the number of mutated sequences, the number of read sequences, the error rate and a predetermined false positive rate.

9. An information processing apparatus, comprising:

a reference nucleic acid base sequence acquisition unit that acquires a reference nucleic acid base sequence including a normal sequence and a mutated sequence;
a sample nucleic acid base sequence acquisition unit that acquires a plurality of sample nucleic acid base sequences read from a sample;
a determination acquisition unit that determines the number of read sequences corresponding to the reference nucleic acid base sequence acquired by the reference nucleic acid base sequence acquisition unit and the number of mutated sequences corresponding to the mutated sequence acquired by the reference nucleic acid base sequence acquisition unit among the sample nucleic acid base sequences acquired by the sample nucleic acid base sequence acquisition unit;
an error rate acquisition unit that acquires an error rate when a nucleic acid base sequence is read; and
a mutation-positive determination unit that determines whether or not the sample nucleic acid base sequence is mutation positive based on the number of mutated sequences and the number of read sequences that are determined by the determination acquisition unit, an error rate acquired by the error rate acquisition unit and a predetermined false positive rate.

EP 3 786 960 A1

FIG. 1

PATIENT

ENDOSCOPY
SURGERY

TREATMENT

SAMPLE

PRETREATMENT

ANALYSIS USING NEXT-
GENERATION SEQUENCER

DETERMINATION OF
GENETIC MUTATION

DECISION FOR
THERAPEUTIC STRATEGY

18

SAMPLE No.1

EXTRACT DNA AND RNA

CANCER
CELL

NORMAL
CELL

DIVIDE

FOR DNA
ANALYSIS

CUT OUT DNA
TARGET SEQUENCE

FOR RNA ANALYSIS

AMPLIFY

AMPLIFY

PURIFY

PURIFY

ADD IDENTIFICATION
ADAPTER

ADD IDENTIFICATION
ADAPTER

PURIFY

PURIFY

MIX

SAMPLE No.2 ······ SAMPLE No.96

ADD DESIGNATION
SEQUENCE

PURIFY

ANALYSIS USING NEXT-
GENERATION SEQUENCER

F I G . 2

# F I G . 3

SAMPLE — TARGET SEQUENCE A — TARGET SEQUENCE B

CUT OUT TARGET SEQUENCE

ADD IDENTIFICATION ADAPTER — IDENTIFICATION ADAPTER — IDENTIFICATION ADAPTER

ADD DESIGNATION SEQUENCE — DESIGNATION SEQUENCE — DESIGNATION SEQUENCE

FIG. 4

REFERENCE SEQUENCE

NORMAL SEQUENCE ······ A T T G C C G C C T C C G A G ······

FIRST MUTATED SEQUENCE ······ A T T G C A G C C T C C G A G ······

SECOND MUTATED SEQUENCE ······ A T T G C T G C C T C C G A G ······

No.                                                    CLASSIFICATION

1  ······ A T T G C C G C C T C C G A G ······ MUTATION NEGATIVE

2  ······ A T T G C C G C C T C C G A G ······ MUTATION NEGATIVE

3  ······ A T T G C A G C C T C C G A G ······ MUTATION POSITIVE (FIRST MUTATED SEQUENCE)

·  ······ A T T G C T G C C T C C G A G ······ MUTATION POSITIVE (SECOND MUTATED SEQUENCE)

·  ······ A T T G C C G C C T C C G A G ······ MUTATION NEGATIVE

MUTATION

READ

·  ······ A A A A G G G G G G G T T T T ······
·  ······ A A A A G G G G G G G T T T T ······   UNRELATED SEQUENCE
·  ······ A A A A G G G G G G G T T T T ······

# FIG.5

MUTATION
NEGATIVE

MUTATION
POSITIVE

BASE SEQUENCE

MUTATION ∼ BASE SEQUENCE

AMPLIFICATION

AMPLIFICATION

AMPLIFICATION

AMPLIFICATION

REPEAT

REPEAT

AMPLIFICATION

AMPLIFICATION

READ BY THE
NEXT-GENERATION
SEQUENCER

READ BY THE
NEXT-GENERATION
SEQUENCER

ATTGCC······

ATTGCA······
↑
MUTATION

22

# F I G . 6

MUTATION
NEGATIVE

BASE SEQUENCE

AMPLIFICATION

AMPLIFICATION

REPEAT

AMPLIFICATION

READ BY THE
NEXT-GENERATION
SEQUENCER

ATTGCC······

MUTATION
POSITIVE

BASE SEQUENCE

AMPLIFICATION

AMPLIFICATION

REPEAT

AMPLIFICATION

ERROR

READ BY THE
NEXT-GENERATION
SEQUENCER

ATTGCA······

ERROR

EP 3 786 960 A1

FIG.7

NUMBER OF MUTATION-POSITIVE SEQUENCES M
[UNIT OF PIECES]

24

EP 3 786 960 A1

FIG. 8

NUMBER OF MUTATION-POSITIVE SEQUENCES M
[UNIT OF PIECES]

# FIG. 9

NEXT-GENERATION SEQUENCER 13

READ DATA FILE 46

FILE SERVER 16

10

INFORMATION PROCESSING APPARATUS 20

CPU 21

MAIN STORAGE DEVICE 22

COMMUNICATION UNIT 24

INPUT UNIT 25

DISPLAY UNIT 26

AUXILIARY STORAGE DEVICE 23

REFERENCE SEQUENCE DB 41

DETERMINATION RESULT DB 43

READING RESULT DB 42

UNKNOWN MUTATION DB 44

FIG. 10

41

| AREA NAME | REFERENCE SEQUENCE | | | | ERROR RATE p |
| | NORMAL SEQUENCE | MUTATED SEQUENCE | | | |
| | | FIRST MUTATED SEQUENCE | SECOND MUTATED SEQUENCE | ······ | |
|---|---|---|---|---|---|
| ALK ex1 | AGCTGCA······ | AGCTGCC······ | AGCTGCT······ | ······ | 0.001 |
| ⋮ | ⋮ | ⋮ | ⋮ | | ⋮ |
| EGFR ex18 | ATGCGAC······ | ATGCGCC······ | ATGCGAA······ | ······ | 0.0025 |
| ⋮ | ⋮ | ⋮ | ⋮ | | ⋮ |
| EML4 ex1 | GGGGCG······ | GGGGCC······ | GGCGCG······ | ······ | 0.0008 |
| ⋮ | ⋮ | ⋮ | ⋮ | | ⋮ |

EP 3 786 960 A1

FIG. 11

SINGLE READ {
@AAAA                          ← READ ID LINE
GCAACTCA··········             ← BASE SEQUENCE LINE
+                              ← + SIGN
C@CCCCEC··········             ← QUALITY LINE
}

@BBBB                          ← READ ID LINE
GATTTGGT··········             ← BASE SEQUENCE LINE
+                              ← + SIGN
!55CCFFBB··········            ← QUALITY LINE
@CCCC                          ← READ ID LINE
TTCAACTC··········             ← BASE SEQUENCE LINE
+                              ← + SIGN
>CCCCC65··········             ← QUALITY LINE

# FIG.12

| SAMPLE ID | A01 |
|---|---|

<u>42</u>

| AREA NAME | READ SEQUENCE NUMBER n | MUTATION-POSITIVE SEQUENCE NUMBER M | | | | |
|---|---|---|---|---|---|---|
| | | FIRST MUTATION-POSITIVE SEQUENCE NUMBER M1 | SECOND MUTATION-POSITIVE SEQUENCE NUMBER M2 | ・・・・・・ | I-th MUTATION-POSITIVE SEQUENCE NUMBER M1 | ・・・・・・ |
| ALK ex1 | 12345 | 0 | 2 | ・・・・・・ | 0 | ・・・・・・ |
| *** | 5888 | 0 | 0 | ・・・・・・ | 5 | ・・・・・・ |
| ⋮ | ⋮ | ⋮ | ⋮ | ⋮ | ⋮ | ⋮ |

EP 3 786 960 A1

# FIG. 13

43

| SAMPLE ID | A01 |
|---|---|

| AREA NAME | MUTATED SEQUENCE | DETERMINATION RESULT | DETERMINATION CONDITION | | |
|---|---|---|---|---|---|
| | | | SPECIFICITY | SENSITIVITY | DETECTION LIMIT VALUE q |
| ALK ex1 | — | ZeroReads | 0.999 | 0.999 | 0.005 |
| ⋮ | ⋮ | ⋮ | ⋮ | ⋮ | ⋮ |
| EGFR ex18 | FIRST MUTATED SEQUENCE | Negative | 0.999 | 0.999 | 0.008 |
| EGFR ex18 | SECOND MUTATED SEQUENCE | Positive | 0.999 | 0.999 | 0.008 |
| ⋮ | ⋮ | ⋮ | ⋮ | ⋮ | ⋮ |

30

# F I G . 14

44

| SAMPLE ID | A01 |
|-----------|-----|

| AREA NAME | READ | REMARKS |
|-----------|------|---------|
| EGFR ex19 | GCATGTGG・・・・・・ | |
| EGFR ex19 | GCATGTGG・・・・・・ | |
| ⋮ | ⋮ | |

FIG. 15

Sample

Analyze:

File:2_S2_L001_R1_001.fastq.gz
2_S2_L001_R2_001.fastq.gz

Reads: 40035
Mapped: 37247
Failed to be mapped: 2788

Germline DNA

| Gene | Allelic Type |
|------|--------------|
| BIM | wt/wt |

Germline RNA

| Gene | Diagnosis | Read # |
|------|-----------|--------|
| OAZ1 | GoodQuality | 7889 |

Somatic mutation DNA

| Area | Diagnosis |
|------|-----------|
| EGFR_ex18 | Negative |
| EGFR_ex19 | Negative |
| EGFR_T790M_Area | Negative |
| EGFR_C797S_Area | Negative |
| EGFR_S768I_Area | Negative |
| EGFR_ex21 | Negative |
| KRAS_ex2 | Negative |
| KRAS_ex3 | Negative |

Message

Somatic mutation RNA

| Area | Diagnosis | Read # |
|------|-----------|--------|
| ALK | Negative | |
| EML4_ALK | Negative | |
| KIF58_ALK | Negative | |
| ALK_others | Negative | |
| RET_fusions | Negative | |
| SDC4_ROS1,S2 | Positive | 237 |

51  52  521  522  53  531  532  533  534

F I G . 1 6

Sample

1
2

Analyze:

File:2_S2_L001_R1_001.fastq.gz
2_S2_L001_R2_001.fastq.gz

Reads: 168948
Mapped: 148669
Failed to be mapped: 20279

Germline DNA

| Gene | Allelic Type |
|------|--------------|
| BIM | wt/wt |

Germline RNA

| Gene | Diagnosis | Read # |
|------|-----------|--------|
| OAZ1 | GoodQuality | 48722 |

Somatic mutation DNA

| Area | Diagnosis | Message |
|------|-----------|---------|
| EGFR_ex18 | Negative | |
| EGFR_ex19 | Negative | |
| EGFR_T790M_Area | Negative | |
| EGFR_C797S_Area | Negative | |
| EGFR_S768I_Area | Negative | |
| EGFR_ex21 | Negative | |
| KRAS_G13C(37G>T) | Positive | 51.6% |
| KRAS_ex3 | Negative | |

Somatic mutation RNA

| Type | Match | ad # |
|------|-------|------|
| KRAS_ex2 | 12254 | |
| KRAS_G12S(34G>A) | 2 | |
| KRAS_G12R(34G>C) | 0 | |
| KRAS_G12C(34G>T) | 2 | |
| KRAS_G12D(35G>A) | 0 | |

Limit of detection: 0.005
Specificity: 0.999
Sensitivity: 0.999

51
52 521 522
53 532 531 535 534

FIG. 17

```
                    ┌──────────┐
                    │  START   │
                    └──────────┘
                         │
                         ▼◄─────────────────────────┐
                  ┌──────────────┐                  │
                  │  INITIALIZE  │ S501             │
                  └──────────────┘                  │
                         │                          │
                         ▼                          │
              ┌─────────────────────┐               │
              │ ACQUIRE REFERENCE   │ S502          │
              │     SEQUENCE        │               │
              └─────────────────────┘               │
                         │                          │
                         ▼◄──────────────────┐      │
              ┌─────────────────────┐        │      │
              │   ACQUIRE A READ    │ S503   │      │
              └─────────────────────┘        │      │
                         │                   │      │
                         ▼                   │      │
              ┌─────────────────────┐        │      │
              │   DETERMINATION     │ S504   │      │
              └─────────────────────┘        │      │
                         │                   │      │
                      S505│                  │      │
                    ◄─────▼─────►    NO       │      │
                   ╱ READ FINISH? ╲──────────┘      │
                    ◄─────┬─────►                   │
                       YES │                        │
                          ▼                         │
              ┌─────────────────────┐               │
              │      RECORD         │               │
              └─────────────────────┘               │
                         │        S506              │
                         ▼                          │
              ┌─────────────────────┐               │
              │     THRESHOLD       │               │
              │   DETERMINATION     │ S507          │
              └─────────────────────┘               │
                         │                          │
                      S508│                         │
                    ◄─────▼─────►     NO            │
                   ╱  REFERENCE  ╲─────────────────┘
                   ╲  SEQUENCE   ╱
                    ◄ FINISH? ──►
                       YES │
                          ▼
                    ┌──────────┐
                    │   END    │
                    └──────────┘
```

34

# FIG. 18

DETERMINATION

S511
COINCIDES WITH A NORMAL SEQUENCE?

NO

YES

S521
I=1

S522
COINCIDE WITH THE I-th MUTATED SEQUENCE?

NO

YES

S523
MUTATED SEQUENCE FINISH?

NO

YES

S524
I=I+1

S526
ADD ONE TO THE NUMBER OF I-th MUTATION-POSITIVE SEQUENCES MI

S527
UNKNOWN MUTATION?

NO

YES

S528
RECORD THE READ

S512
ADD ONE TO THE NUMBER OF READ SEQUENCES n

RETURN

FIG.19

THRESHOLD
DETERMINATION

ACQUIRE INITIAL
CONDITIONS — S531

S532
n=0? — NO

YES

DETERMINATION
RESULT = "ZeroReads" — S533

RECORD — S534

RETURN

FIRST THRESHOLD
MP CALCULATION — S541

SECOND THRESHOLD
MN CALCULATION — S542

S543
$MP \leqq MN$? — NO

YES

STATISTICAL
DETERMINATION — S544

RETURN

S545
DETECTION
LIMIT VALUE q MAY
BE INCREASED? — NO

YES

INCREASE DETECTION
LIMIT VALUE q — S546

DETERMINATION RESULT
= "not determined" — S583

RECORD — S584

RETURN

FIG.20

STATISTICAL
DETERMINATION

I=1 — S551

ADDS NEW RECORD — S552

ACQUIRE I-th
MUTATION-POSITIVE
SEQUENCE NUMBER MI — S553

S554
MI≦MN?

NO

YES

S556
DETERMINATION RESULT
= "Positive"

DETERMINATION RESULT
= "Negative" — S555

RECORD — S557

S558
FINISH?

NO

YES

S559
I=I+1

RETURN

# FIG. 21

```
    ┌─────────────────────┐
    │   FIRST THRESHOLD   │
    │   MP CALCULATION    │
    └─────────────────────┘
              │
    ┌─────────────────┐  S561
    │      F = 0      │
    └─────────────────┘
              │
    ┌─────────────────┐  S562
    │      M = 1      │
    └─────────────────┘
              │
    ┌─────────────────┐  S563
    │ CALCULATE f(n, M, p) │
    └─────────────────┘
              │
    ┌─────────────────┐  S564
    │  ADD f(n, M, p) TO F │
    └─────────────────┘
              │
            S565
        ◇ F ≧ SPECIFICITY? ◇── NO ──┐
          YES │                      │  S566
              │              ┌───────────────┐
              │              │ ADD ONE TO M  │
              │              └───────────────┘
              │
    ┌─────────────────────┐  S567
    │ FIRST THRESHOLD MP = M │
    └─────────────────────┘
              │
         ┌──────────┐
         │  RETURN  │
         └──────────┘
```

# FIG.22

F I G . 23

| LIMIT OF DETECTION VALUE q | SENSITIVITY | SPECIFICITY |
|---|---|---|
| 0.005 | 0.999 | 0.999 |
| 0.006 | 0.999 | 0.999 |
| 0.007 | 0.999 | 0.999 |
| 0.008 | 0.999 | 0.999 |
| 0.009 | 0.999 | 0.999 |
| 0.01 | 0.99 | 0.99 |
| 0.02 | 0.99 | 0.99 |
| 0.03 | 0.99 | 0.99 |
| 0.04 | 0.99 | 0.99 |
| 0.05 | 0.98 | 0.98 |
| 0.06 | 0.98 | 0.98 |
| 0.07 | 0.98 | 0.98 |
| 0.08 | 0.98 | 0.98 |
| 0.09 | 0.98 | 0.98 |
| 0.1 | 0.95 | 0.95 |
| 0.2 | 0.95 | 0.95 |
| 0.3 | 0.95 | 0.95 |
| 0.4 | 0.95 | 0.95 |
| 0.5 | 0.95 | 0.95 |

FIG. 24

```
        ┌─────────────────────┐
        │     THRESHOLD       │
        │   DETERMINATION     │
        └──────────┬──────────┘
                   │
        ┌──────────┴──────────┐
        │  ACQUIRE INITIAL    │ S531
        │    CONDITIONS       │
        └──────────┬──────────┘
                   │
              ╱────┴────╲  S532        NO
             ╱   n=0?    ╲──────────────────────────┐
             ╲           ╱                          │
              ╲────┬────╱                           │
               YES │                                │
        ┌──────────┴──────────┐        ┌───────────┴─────────┐
        │   DETERMINATION     │ S533   │  FIRST THRESHOLD    │ S541
        │ RESULT = "ZeroReads"│        │   MP CALCULATION    │
        └──────────┬──────────┘        └───────────┬─────────┘
                   │                                │
        ┌──────────┴──────────┐        ┌───────────┴─────────┐
        │      RECORD         │ S534   │  SECOND THRESHOLD   │ S542
        └──────────┬──────────┘        │   MN CALCULATION    │
                   │                   └───────────┬─────────┘
            ╭──────┴──────╮                        │
            │   RETURN    │                   ╱────┴────╲  S543     NO
            ╰─────────────╯                  ╱  MP≦MN?   ╲──────────┐
                                             ╲           ╱          │
                                              ╲────┬────╱           │
                                               YES │                │
                                     ┌─────────────┴───────┐        │
                                     │    STATISTICAL      │ S544   │
                                     │   DETERMINATION     │        │
                                     └─────────────┬───────┘        │
                                                   │                │
                                            ╭──────┴──────╮         │
                                            │   RETURN    │         │
                                            ╰─────────────╯         │
                                                          ┌─────────┴─────────┐
                                                          │    PARAMETER      │ S581
                                                          │   ADJUSTMENT      │
                                                          └─────────┬─────────┘
                                                                    │
                                                   NO         ╱─────┴─────╲  S582
                                              ┌─────────────╱ PARAMETER MAY BE╲
                                              │             ╲    CHANGED?     ╱
                                              │              ╲─────┬─────╱
                                              │                YES │       S585
                                              │               ╱────┴────╲       NO
                                              │              ╱ SPECIFICITY ╲───────┐
                                              │              ╲ MAY BE       ╱      │
                                              │              ╲ CHANGED?    ╱       │
                                              │               ╲────┬────╱          │
                                              │               YES  │               │
                                 ┌────────────┴─────────┐          │               │
                                 │ DETERMINATION RESULT │ S583     │               │
                                 │  = "not determined"  │          │               │
                                 └────────────┬─────────┘          │               │
                                              │                                    │
                                 ┌────────────┴─────────┐                          │
                                 │       RECORD         │ S584                     │
                                 └────────────┬─────────┘                          │
                                              │                                    │
                                       ╭──────┴──────╮                             │
                                       │   RETURN    │                             │
                                       ╰─────────────╯                             │
```

41

# FIG. 25

```
        ┌─────────────────┐
        │   PARAMETER      │
        │   ADJUSTMENT     │
        └─────────────────┘
                 │
                 ▼         S601
        ┌─────────────────┐
        │ STORE PARAMETERS │
        └─────────────────┘
                 │
                 ▼         S602
        ┌─────────────────┐
        │  EXTRACT RECORD  │
        └─────────────────┘
                 │
                 ▼         S603
              ╱REDUCED╲              NO
           ╱ SENSITIVITY IS ╲──────────────────┐
            ╲ SELECTABLE? ╱                     │
              ╲       ╱                          │
            YES │                                │
                │         S604                   ▼          S605
    ┌────────────────────┐            ┌─────────────────┐
    │ SELECTS THE RECORD │            │  EXTRACT RECORD  │
    │ INCLUDING REDUCTION│            └─────────────────┘
    │   OF SPECIFICITY   │                     │
    └────────────────────┘                     ▼         S606
                │                           ╱REDUCED╲            NO
                │                        ╱ SENSITIVITY IS ╲──────────────┐
                │                         ╲ SELECTABLE? ╱                 │
                │                           ╲       ╱                     │
                │                         YES │                           │
                │                             │         S607              │
                │                 ┌────────────────────┐                  │
                │                 │ SELECTS THE RECORD │                  │
                │                 │ INCLUDING REDUCTION│                  │
                │                 │   OF SENSITIVITY   │                  │
                │                 └────────────────────┘                  │
                │                             │                           │
                │                             │              S608         ▼
                │                             │        ┌─────────────────┐
                │◄────────────────────────────┘        │  EXTRACT RECORD  │
                │                                       └─────────────────┘
                │                                               │
                │                                               ▼       S609
                │                                      ╱  DETECTION ╲
                │              NO                     ╱  LIMIT VALUE q MAY ╲
                │◄───────────────────────────────────╲  BE INCREASED? ╱
                │                                       ╲           ╱
                │                                          YES │
                │                                              │    S610
                │                                   ┌─────────────────┐
                │                                   │ INCREASE DETECTION│
                │                                   │  LIMIT VALUE q   │
                │                                   └─────────────────┘
                │                                              │
                │◄─────────────────────────────────────────────┘
                │
                ▼
        ┌─────────────────┐
        │     RETURN       │
        └─────────────────┘
```

# FIG. 26

```
          ┌─────────┐
          │  START  │
          └────┬────┘
               │
     ┌─────────▼──────────┐
     │ EXTRACT RECORDS OF │  S621
     │    ONE SAMPLE      │
     └─────────┬──────────┘
               │
     ┌─────────▼──────────┐
     │ EXTRACTS FIRST GROUP│  S622
     └─────────┬──────────┘
               │           S623
         ◇─────────────◇
   NO ◇    DETECT?     ◇
     ┌──◇             ◇
     │    ◇─────────◇
     │        │YES
     │                  S624
     │    ◇─────────────◇     NO
     │  ◇   MISSING?     ◇──────┐
     │    ◇             ◇       │
     │      ◇─────────◇         │
     │        │YES              │
     │  ┌─────▼──────────────┐  │
     │  │ MISSING READ IS    │  │ S625
     │  │    "Negative"      │  │
     │  └─────┬──────────────┘  │
     └────────┤◄────────────────┘
              │
              │            S626
        ◇─────────────────◇
   NO ◇  EGFR ex20 HAS    ◇
     ┌◇  T790M MUTATION?   ◇
     │  ◇                 ◇
     │    ◇─────────────◇
     │        │YES
     │                    S627
     │    ◇─────────────◇     NO
     │  ◇   EGFR ex19     ◇──────┐
     │  ◇  IS MISSING?    ◇      │
     │    ◇             ◇        │
     │      ◇─────────◇          │
     │        │YES               │
     │  ┌─────▼──────────────┐   │
     │  │ EGFR ex19 IS       │   │ S628
     │  │    "Positive"      │   │
     │  └─────┬──────────────┘   │
     │        │◄─────────────────┘
     │        │           S629
     │    ◇─────────────◇     NO
     │  ◇ L858R IS MISSING? ◇────┐
     │    ◇             ◇        │
     │      ◇─────────◇          │
     │        │YES               │
     │  ┌─────▼──────────────┐   │
     │  │ L858R IS "Positive"│   │ S630
     │  └─────┬──────────────┘   │
     └────────┤◄─────────────────┘
              │
           ┌──▼──┐
           │  1  │
           └─────┘
```

# F I G . 27

```
                    ( 1 )
                      │
                      ▼
        ┌──────────────────────────────┐  S641
        │  ACQUIRE NORMAL SEQUENCE OF   │
        │          EGFR ex19            │
        └──────────────────────────────┘
                      │
    ┌─────────────────┤
    │                 ▼
    │     ┌──────────────────────────────┐  S642
    │     │        EXTRACT RECORD         │
    │     └──────────────────────────────┘
    │                 │
    │                 ▼        S643
    │              ◇─────────◇
    │            ◇             ◇    NO
    │          ◇  DELETION IN    ◇──────────┐
    │            ◇  EGFR ex19?  ◇           │
    │              ◇─────────◇              │
    │                 │                     │
    │                YES                    │
    │                 ▼        S644         │
    │              ◇─────────◇              │
    │            ◇  NUMBER OF   ◇           │
    │          ◇  DELETED BASES IS ◇   NO   │
    │          ◇   MULTIPLE OF 3?  ◇────────┤
    │            ◇             ◇            │
    │              ◇─────────◇              │
    │                 │                     │
    │                YES                    │
    │                 ▼                     │
    │     ┌──────────────────────────────┐  S645
    │     │  NEW EGFR ex19 WITH DELETION  │   │
    │     └──────────────────────────────┘   │
    │                 │◄────────────────────┘
    │                 ▼        S646
    │     NO       ◇─────────◇
    └────────────◇   FINISH?   ◇
                   ◇─────────◇
                      │
                    YES
                      ▼
                    ( 2 )
```

F I G . 28

```
                            ┌───┐
                            │ 2 │
                            └─┬─┘
                              ↓
              ┌──────────────────────┐  S651
              │   ACQUIRE NORMAL      │
              │   SEQUENCE OF ALK     │
              └──────────┬───────────┘
                         ↓
    ┌──────────→┌──────────────────────┐  S652
    │           │    EXTRACT RECORD     │
    │           └──────────┬───────────┘
    │                      ↓            S653
    │                     ╱ ╲              NO
    │          FUSED GENE OF ALK AND X? ────────────┐
    │                     ╲ ╱                        │
    │                   YES↓                         │
    │                      ↓            S654         │
    │                     ╱ ╲              NO         │
    │          FUSED AT ACCEPTOR SITE? ─────────────→│
    │                     ╲ ╱                        │
    │                   YES↓                         │
    │                      ↓            S655         │
    │                     ╱ ╲              NO         │
    │          READING FRAME COINCIDE? ─────────────→│
    │                     ╲ ╱                        │
    │                   YES↓                         │
    │           ┌──────────────────────┐  S656       │
    │           │  NEW ALK FUSION GENE  │            │
    │           └──────────┬───────────┘            │
    │                      ↓←──────────────────────┘
    │                      ↓            S657
    │         NO          ╱ ╲
    └────────────────── FINISH?
                         ╲ ╱
                       YES↓
                    ┌─────────┐
                    │   END   │
                    └─────────┘
```

F I G . 29

<u>20</u>

| REFERENCE NUCLEIC ACID BASE SEQUENCE ACQUISITION UNIT | 81 |

| SAMPLE NUCLEIC ACID BASE SEQUENCE ACQUISITION UNIT | 82 |

| DETERMINATION ACQUISITION UNIT | 83 |

| ERROR RATE ACQUISITION UNIT | 84 |

| MUTATION-POSITIVE DETERMINATION UNIT | 85 |

# FIG. 30

10

16

NEXT-GENERATION
SEQUENCER
13

FILE SERVER

READ
DATA FILE
46

97

96

90

COMPUTER

CPU
21

MAIN STORAGE
DEVICE
22

COMMUNICATION
UNIT
24

READING
UNIT
27

98

33

AUXILIARY STORAGE DEVICE

INPUT UNIT
25

DISPLAY
UNIT
26

REFERENCE
SEQUENCE DB
41

DETERMINATION
RESULT DB
43

READING
RESULT DB
42

UNKNOWN
MUTATION DB
44

**EP 3 786 960 A1**

<table>
<tr><td colspan="2">**INTERNATIONAL SEARCH REPORT**</td><td>International application No.<br>PCT/JP2019/012294</td></tr>
</table>

A. CLASSIFICATION OF SUBJECT MATTER
Int.Cl. G16B30/00(2019.01)i, C12M1/00(2006.01)i, C12Q1/6809(2018.01)i

According to International Patent Classification (IPC) or to both national classification and IPC

B. FIELDS SEARCHED

Minimum documentation searched (classification system followed by classification symbols)
Int.Cl. G16B30/00, C12M1/00, C12Q1/6809

Documentation searched other than minimum documentation to the extent that such documents are included in the fields searched
Published examined utility model applications of Japan    1922–1996
Published unexamined utility model applications of Japan    1971–2019
Registered utility model specifications of Japan    1996–2019
Published registered utility model applications of Japan    1994–2019

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2017-212938 A (FUJIFILM CORPORATION) 07 December 2017, entire text, all drawings (Family: none) | 1-9 |
| A | JP 2017-520821 A (F. HOFFMANN-LA ROCHE AG) 27 July 2017, entire text, all drawings<br>& US 2015/0324519 A1 & WO 2015/173222 A1 & EP 3143537 A1 & CN 106462670 A | 1-9 |

☐ Further documents are listed in the continuation of Box C.        ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>12.06.2019 | Date of mailing of the international search report<br>25.06.2019 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

48

**REFERENCES CITED IN THE DESCRIPTION**

**Patent documents cited in the description**

- JP 2016086678 A